# EUROPEAN PATENT APPLICATION

(11) **EP 3 308 705 A1**
(43) Date of publication of application: **18.04.2018**
(21) Application number: 17195915.8
(22) Date of filing: 11.10.2017
(51) Int. Cl.: A61B 5/053

(54) **BODY COMPOSITION MEASURING DEVICE**

(30) Priority: 12.10.2016 KR 20160132277
(71) Applicant: LG Electronics Inc., Seoul 07336 (KR)
(72) Inventor: YI, Ilbyoung, 06772 Seoul (KR); HAN, Sehyun, 06772 Seoul (KR); PARK, Jeongsoo, 06772 Seoul (KR); KIM, Hyungsun, 06772 Seoul (KR); PARK, Jaewan, 06772 Seoul (KR)
(74) Representative: Katérle, Axel

(57) **Abstract**

A body composition measuring device including a body portion including first and second sides facing each other and a lateral side connecting the first and second sides; a first electrode portion provided on the first side of the body portion; a second electrode portion on the second side of the body portion; and a control part that measures an impedance when the first and second electrode portions come into contact with first and second areas of a user holding the body composition measuring device.

## Description

### BACKGROUND OF THE INVENTION

### 1. Field of the Invention

The present invention relates to a body composition measuring device which measures body composition when in contact with a human body, and a method of controlling the same.

### 2. Description of the Related Art

There is a growing interest in healthcare, along with the progress in medical science and the increase in average life expectancy. In connection with this, the interest in medical devices is rising too. The scope of medical devices is being expanded to include medium- to small-sized medical devices kept in public institutions, etc. or small-sized medical devices individuals can possess or carry, as well as a variety of medical devices used in hospitals or healthcare institutions.

A body composition measuring device, which is a type of healthcare device, measures body composition by bioelectrical impedance analysis (BIA). In BIA, the human body is viewed as a combination of impedances, and a current is applied to the human body, a voltage resulting from bioelectrical impedance is measured, and then the bioelectrical impedance is determined using this current and voltage.

One disadvantage of BIA is that it is hard to use mobile devices to measure body composition since multiple electrodes require contact with extremities of the human body to provide an accurate measure for different areas of the body, so it cannot keep up with the needs of people having a lot of interest in health who want to get their body composition measured without visiting hospitals or medical check-up centers.

### SUMMARY OF THE INVENTION

The present invention has been made in an effort to provide a mobile device which measures body composition data of different parts of a human body, and a method of controlling the same.

An exemplary embodiment of the present invention provides a body composition measuring device including: a body portion including first and second sides facing each other and a lateral side connecting the first and second sides; first and second electrode portions provided on the first and second sides; and a control part that measures impedance when the first and second electrode portions come into contact with first and second areas of a human body. Accordingly, the user can obtain the impedance of a part of the body by bringing each body region into contact with the two sides.

In one embodiment, the body composition measuring device may further include a length measuring portion that is provided on the first and second bodies and exposed to outside by external force to enable measurement of the length and circumference of a part of the body, or a sensing part that senses rotation of the body portion, whereby the length and circumference of a desired part of the body may be measured.

In one embodiment, in simple mode, the control part may obtain an impedance measurement corresponding to a single posture while the first and second electrode portions are in contact with the human body, and in complete mode, the control part may obtain a plurality of impedance measurements corresponding to a plurality of postures while the first and second electrode portions are in contact with the human body, whereby the user can obtain body measurement data in a desired mode when necessary.

Another exemplary embodiment of the present invention provides a mobile terminal which includes an electrode portion located on both sides of a body portion and performs wireless communication with a body composition measuring device that obtains an impedance measurement when a human body comes into contact with the electrode portion, the mobile terminal including: a display; a wireless communication part that performs wireless communication with the body composition measuring device; and a control part that receives an impedance measurement from the body composition measuring device and that displays a body composition measurement result screen for the impedance measurement on the display.

In one embodiment, the mobile terminal may further include a memory for storing the body composition measurement data, wherein the body composition measurement result screen may include at least one photograph captured of the user, and the memory may store the at least one photograph along with the body composition measurement data, whereby the user can see calculated measurement data along with photographs of the body.

In one embodiment, if the measurement data is out of a preset reference range, the control part controls the display to display screen information for changing preset user information, and the control part outputs at least one photograph of a person extracted based on the measurement result data to the screen information, whereby a person who had their body composition measured may be correctly assigned as the user.

The body composition measuring device according to an embodiment of the present invention allows the user to easily bring an electrode into contact with each area of the body since the electrode to be brought into contact with the body is mounted on both sides of the body portion. Moreover, a body composition measurement result may be provided with an accuracy level desired by the user since body composition may be measured in simple mode and complete mode.

Moreover, the length and circumference of a part of the body can be measured by rotating the body portion with a circular cross-section, thereby allowing the user to obtain a more accurate body measurement result.

The mobile terminal according to an embodiment of the present invention can provide a measurement result of a level desired by the user by operating the body composition measuring device in complete mode or in simple mode.

Further, body composition measurement data is provided along with a captured image, and if the measurement data does not match the existing body information of the user, another user matching the measurement result data may be recommended to provide more accurate measurement data.

Further scope of applicability of the present invention will become apparent from the detailed description given hereinafter. However, the detailed description and specific examples, while indicating preferred embodiments of the invention, are given by illustration only, since various changes and modifications within the spirit and scope of the invention will become apparent to those skilled in the art from this detailed description.

### BRIEF DESCRIPTION OF THE DRAWINGS

The accompanying drawings, which are included to provide a further understanding of the invention and are incorporated in and constitute a part of this specification, illustrate exemplary embodiments and together with the description serve to explain the principles of the invention.

In the drawings:
FIG. 1A is a conceptual diagram of a body composition measuring device according to one embodiment of the present invention when viewed from one direction, and FIG. 1B is a conceptual diagram of the shape of electrode portions of a body composition measuring device according to another embodiments of the present invention;
FIG. 1C is a cross-sectional view of the body composition measuring device according to one embodiment of the present invention, and FIG. 1D is a block diagram explaining the components of the body composition measuring device according to an embodiment of the present invention;
FIGS. 2A to 2D are views of a body composition measuring device according to another embodiment of the present invention when viewed from one direction;
FIGS. 3A to 3C are views of body composition measuring devices according to further embodiments of the present invention when viewed from one direction;
FIG. 4A is a conceptual diagram explaining a method of estimating total body impedance using impedance measurements of different areas of the human body, obtained by the body composition measuring device of the present invention;
FIG. 4B is a conceptual diagram explaining a control method for measuring the circumference/length of a part of the human body in order to create body composition measurement data;
FIG. 4C is a conceptual diagram explaining how the light-emitting regions in the output part emit light according to the measurement level;
FIG. 4D is a conceptual diagram explaining the components of an example of an external device which can be connected wirelessly to the body composition measuring device 100 according to an embodiment of the present invention;
FIGS. 5A to 5E are conceptual diagrams explaining a control method for a mobile terminal that communicates wirelessly with the body composition measuring device according to one embodiment;
FIGS. 6A to 6C are conceptual diagrams explaining the mobile terminal performing the measurement function and a control method for the body composition measuring device;
FIGS. 7A and 7B are conceptual diagrams explaining a control method for giving a notification of measurement by the body composition measuring device;
FIGS. 8A and 8B are conceptual diagrams explaining a control method for providing a measurement result obtained by the body composition measuring device;
FIG. 8C is a conceptual view illustrating a measurement result screen which displays a body fat ratio (percentage) and the number of steps;
FIG. 8D is a conceptual view illustrating an eighth measurement result screen indicating a change of a weight;
FIGS. 9A and 9B are conceptual diagrams explaining a method of controlling result data according to one embodiment of the present invention;
FIGS. 10A to 10D are conceptual diagrams explaining a control method for creating target data using a measurement result screen;
FIG. 10E is a conceptual view illustrating a target setting screen which explains a target value.
FIGS. 11A to 11D are conceptual diagrams explaining a control method for storing photographic data along with result data according to one embodiment;
FIGS. 12A and 12B are conceptual diagrams explaining a control method for identifying a person for whom the body composition measuring device 100 has obtained a measurement result;
FIGS. 13A and 13B are conceptual diagrams explaining a control method for sharing measurement data; and
FIG. 13C is a conceptual diagram explaining a method of providing a screen of an analysis result of shared measurement data;
FIG. 13D is a conceptual view illustrating a control method to display a distribution graph in a filtering manner;
FIG. 14 is a conceptual diagram explaining a method of controlling a body composition measurement and motion data according to one embodiment; and
FIGS. 15A to 15B are conceptual views illustrating a method to control an application execution screen.

### DETAILED DESCRIPTION OF THE INVENTION

Description will now be given in detail according to exemplary embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. The accompanying drawings are used to help easily understand various technical features and it should be understood that the embodiments presented herein are not limited by the accompanying drawings. As such, the present disclosure should be construed to extend to any alterations, equivalents and substitutes in addition to those which are particularly set out in the accompanying drawings.

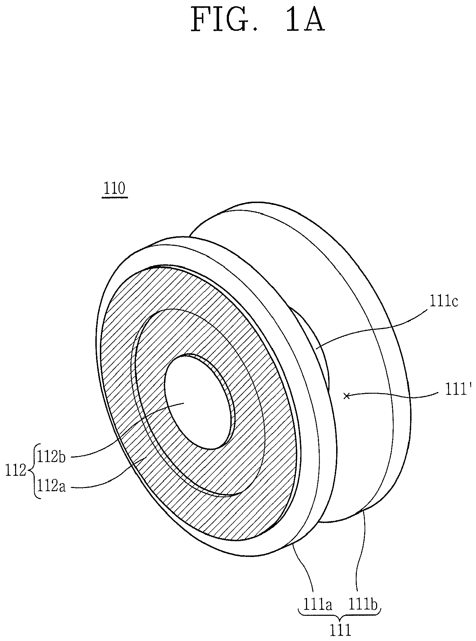

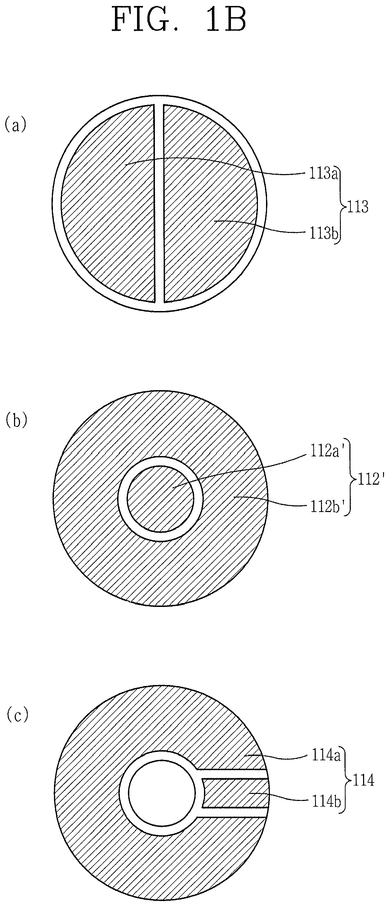

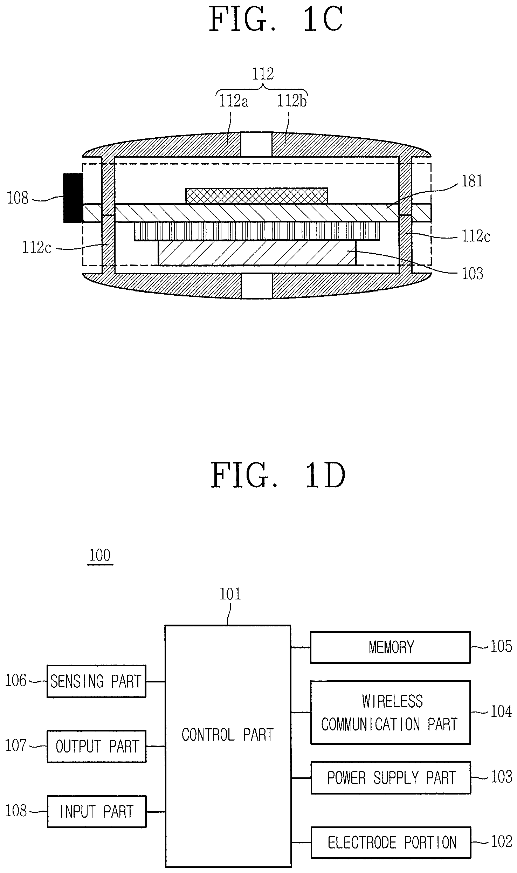

FIG. 1A is a conceptual diagram of a body composition measuring device 100 according to one embodiment of the present invention when viewed from one direction, and FIG. 1B is a conceptual diagram of the shape of electrode portions of a body composition measuring device 100 according to other embodiments of the present invention. Further, FIG. 1C is a cross-sectional view of the body composition measuring device 100 according to one embodiment of the present invention, and FIG. 1D is a block diagram explaining the components of the body composition measuring device 100 according to an embodiment of the present invention.

Referring to FIG. 1A, a first body composition measuring device 110 according to this embodiment includes a body portion 111 including first to third bodies 111a, 111b, and 111c and an electrode module. The body portion 111 is made up of insulating members. Preferably, the body portion 111 of the first body composition measuring device 110 is a sufficient size for holding in both hands. That is, a cross-section of the first and second bodies 111a and 111b may be palm-size.

The first and second bodies 111a and 111b are of equal size, positioned to face each other, and connected by the third body 111c. The first and second bodies 111a and 111b constitute first and second sides of the body portion 111 facing each other. The third body 111c connects the first and second bodies 111a and 111c to form the lateral side of the body portion 111. A cross-section of the third body 111c is narrower than a cross-section of the first and second bodies 111a and 111b. That is, a space 111' may be formed between the first and second bodies 111a and 111b.

A first electrode portion 112 and a second electrode portion are respectively provided on the first and second sides of the body portion 111, which are formed by the first and second bodies 111a and 111b. The first electrode portion 112 and the second electrode portion may have substantially the same shape. For example, the first electrode portion 112 includes first and second electrodes 112a and 112b.

The first electrode 112a is in the shape of a closed loop, adjacent to the edge of the first side, and the second electrode 112b is located at the center of the first side. The first and second electrodes 112a and 112b are spaced apart from each other, and the first electrode 112a can surround the second electrode 112b but the shape of the electrodes of an electrode portion are not limited to this.

The first and second electrodes 112a and 112b preferably protrude from the first side so as to make it easy for the first and second electrodes 112a and 112b to come into contact with the palm of the user's hand when the user touches the body composition measuring device 110 on the palm.

Referring to FIG. 1B, the electrodes of an electrode portion include a variety of shapes. An electrode portion 113 of (a) of FIG. 1B includes first and second electrodes 113a and 113b that are spaced apart from each other and symmetrical in shape. The first and second electrodes 113a and 113b easily come into contact with the human body since they take up most of the surface area of the first side.

An electrode portion 112' of (b) of FIG. 1B includes a first electrode 112a' provided in the center and a second electrode 112b' shaped to surround the first electrode 112a'. The space between the first and second electrodes 112a' and 112b' of (b) of FIG. 1B can be narrower compared to that between the first and second electrodes 112a and 112b of FIG. 1A. In this instance, the electrode portion may be larger in size, thus ensuring that the surface area contacting the human body is as large as possible.

An electrode portion 114 of (c) of FIG. 1B includes a first electrode 114a forming a closed loop around the center and a second electrode 114b located between both ends of the first electrode 114a. The body composition measuring device 100 including the electrode portion 114 according to this embodiment can guide a certain finger to the second electrode 114b so that the finger is correctly positioned for measurement. The user can deliberately bring the second electrode 114b into contact with some of their fingers so that it easily comes into contact with the human body. The shape of the second electrode 114b is not limited to what is described above.

As shown in FIG. 1B, electrode portions can come in a variety of shapes, and includes one electrode or a plurality of electrodes. In addition, the electrode portions provided on the first and second sides may be, but not limited to, symmetrical in shape. Since a first electrode and a second electrode, an input electrode and an output electrode, are disposed on one surface of the body portion 111 of the body composition measuring device 100, a part of a human body having a relatively wide area, such as the palm and the leg, can be measured.

Referring to FIG. 1C, the body composition measuring device 100 according to one embodiment of the present invention may further include a circuit board 181 located inside the body portion 111, a battery 103 that supplies power to the body composition measuring device 100, and a switch part 108 for switching the power mode of the body composition measuring device 100 or applying a control command. Moreover, memory and various chip structures for calculating body composition measurement data may be located inside the body portion 111.

The circuit board 181 may be set substantially parallel to the first and second sides. The body composition measuring device 100 further includes a connecting portion 112c that extends into the body portion from the first and second electrodes 112a and 112b and that is electrically connected to the circuit board 181. Thus, the first electrode portion 112 and second electrode portion making contact with the human body can be electrically connected to the circuit board 181.

Referring to FIG. 1D, the body composition measuring device 100 according to one embodiment of the present invention includes a control part 101, a sensing part 106 including at least one sensor, an output part 107, an input part 108, a memory 105, a wireless communication part 104, a power supply part 103, and an electrode portion 102.

The sensing part 106 may include one or more sensors for sensing at least either information on the environment surrounding the body composition measuring device 100 or user information. If desired, the sensing unit 140 may alternatively or additionally include other types of sensors or devices, such as a proximity sensor 141, an illumination sensor 142, a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 121), a microphone 122, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The mobile terminal may be configured to utilize information obtained from sensing unit 140, and in particular, information obtained from one or more sensors of the sensing unit 140, and combinations thereof.

The output part 107 may include at least one among a light-emitting part for emitting light based on the operation of the measuring device 100, a display for outputting visual information, an audio output part for outputting auditory information, and a haptic module for producing vibrations. The input part 108 may be implemented as a key switch for receiving physical input, a touch sensor for receiving touch input, etc., in order to receive control commands. The memory 105 may save changes in measured impedance, movement data sensed by the sensing part 106, and body composition data calculated from the measured impedance.

The wireless communication part 104 performs wireless communication with a particular external device. The particular external device is a mobile terminal 200, and sends and receives the measured impedance or the calculated body composition data. The electrode portion 102 includes a variety of shapes according to various embodiments, as shown in FIGS. 1A and 1B.

The control part 101 can control the wireless communication part 104 to create body composition measurement data using the impedance measured by the electrode portion 102 or to send the impedance to the particular external device. Moreover, the input part 108 may include a fingerprint sensor for sensing an input device for sensing fingerprints or an input device for entering a password. When a fingerprint or password is authenticated, the body composition measuring device may go into operation, or the fingerprint or password may be identified and measurement data may be created using the user information.

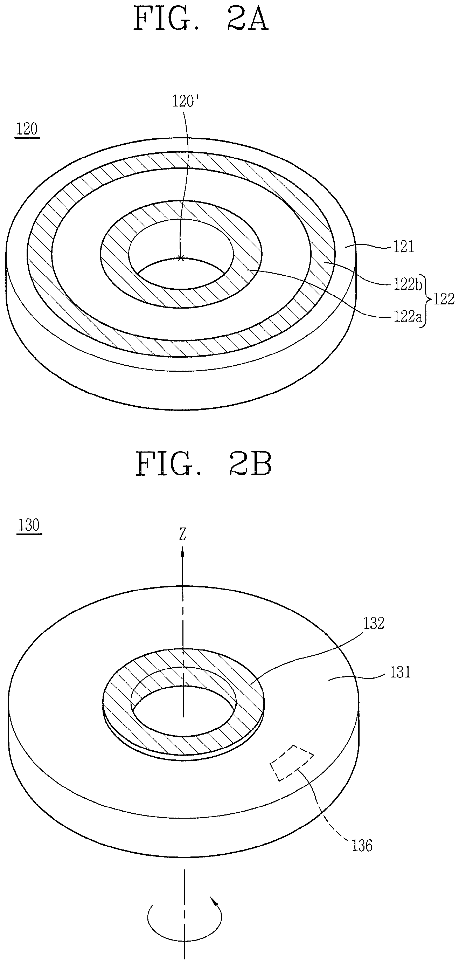

Next, FIGS. 2A to 2D are views of a body composition measuring device 100 according to another embodiment of the present invention when viewed from one direction. As shown, the body composition measuring device 120 of FIG. 2A includes a body portion 121 and an electrode portion 122. The body portion 121 has a recess 120' at the center. Further, the electrode portion 122 includes first and second electrodes 122a and 122b. The electrode portion 122 is also provided on both first and second sides of the body portion 121 facing each other.

In addition, the first electrode 122a surrounds the recess 120', and the second electrode 122b is in the shape of a closed loop surrounding the first electrode 122a. The first and second electrodes 122a and 122b are also spaced apart from each other, and the electrode portions on the first and second sides may be, but not limited to, symmetrical. The body portion 121 may be composed of a single unit, and the side of the body portion 121 may include one surface.

The body composition measuring device 130 of FIG. 2B includes a body portion with a recess at the center and an electrode portion 132 provided on the body portion 131. The electrode portion 132 is provided on a first side of the body portion 131, and extends to the inside of the body portion 131 forming the recess. An electrode portion having a shape different from that of the electrode portion 132 on the first side can also be provided on a second side of the body portion 131.

Thus, the body portion 131 can be easily held and rotated by inserting the user's finger or the like into the recess. The body composition measuring device 130 according to this embodiment further includes a sensor part 136 for sensing rotation of the body portion 131. In addition, the sensor part 136 may be implemented as a geomagnetic sensor, acceleration sensor, or gyro sensor which senses rotation and movement of the body portion 131. For example, when the body portion 131 moves along the circumference of the user's arm while rotating, the control part 101 can measure the circumference of the arm by the rotation of the body portion 131. Circumference and/or distance may be calculated based on the outer circumference of the body portion 131 and the rotation of the body portion 131.

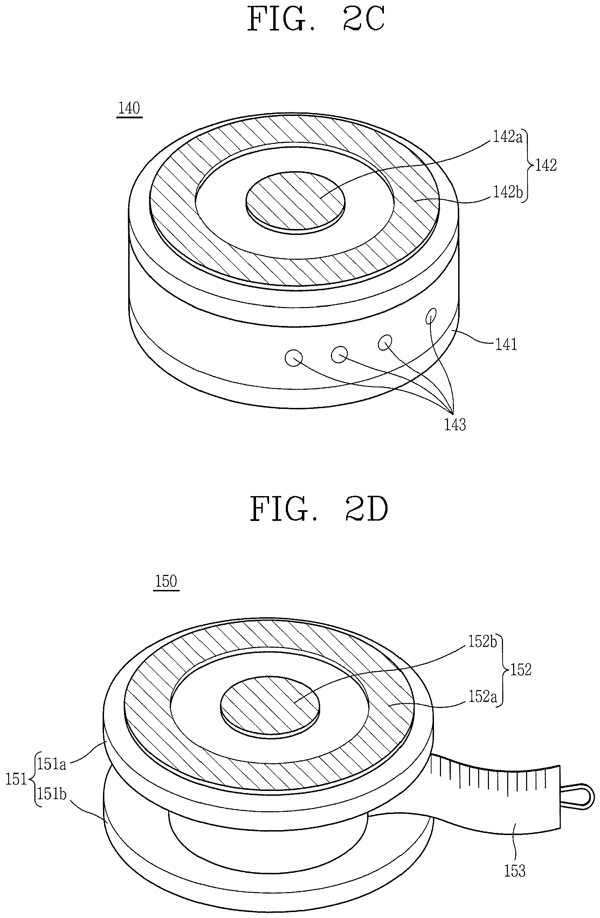

In addition, body composition measuring device 140 of FIG. 2C includes a body portion 141, an electrode portion 142, and a light-emitting portion 143. The body portion 141 includes first and second sides facing each other which are configured to come into contact with an area of the human body. The electrode portion 142 is formed on both of the first and second sides. The electrode portion 142 includes first and second electrodes 142a and 142b. The first electrode 142a may be provided at the center of the first side, and the second electrode 142b may surround the first electrode 142a and run along the edge of the first side. The first and second electrodes 142a and 142b are spaced apart from each other.

The light-emitting portion 143 may have light-emitting regions for emitting light in different colors, which vary in number depending on the execution of the body composition measurement function, the mode of the body composition measurement function, and the level of the body composition measurement function. The light-emitting portion 143 may include LED elements, and the positions of the LED elements on the body portion 141 are not limited to what is illustrated in the figure.

The body composition measuring device 150 of FIG. 2D includes a body portion 151, an electrode portion 152, and a length measuring portion 153. The body portion 151 of the body composition measuring device 100 according to this embodiment includes first and second bodies 151a and 151b facing each other, and the first and second bodies 151a and 151c may be connected by a connecting portion. The length measuring portion 153 may be wound between the first and second bodies 151a and 151b.

The length measuring portion 153 may be in the form of measuring tape that may change shape as it unwinds from the connecting portion by external force and gets exposed. The length measuring portion 153, after use, may be rewound and held in place on the body portion 151 by an elastic force from an elastic portion located outside the body portion 151. The circumference or length of a part of the human body may be measured by means of the length measuring portion 153.

The electrode portion 152 includes first and second electrodes 152a and 152b spaced apart from each other, and the shape of the first and second electrodes 152a and 152b are not limited to what is illustrated in FIG. 2D. The electrode portion 152 is positioned to correspond to the outer sides of the first and second bodies 151a and 151b which are opposite to each other.

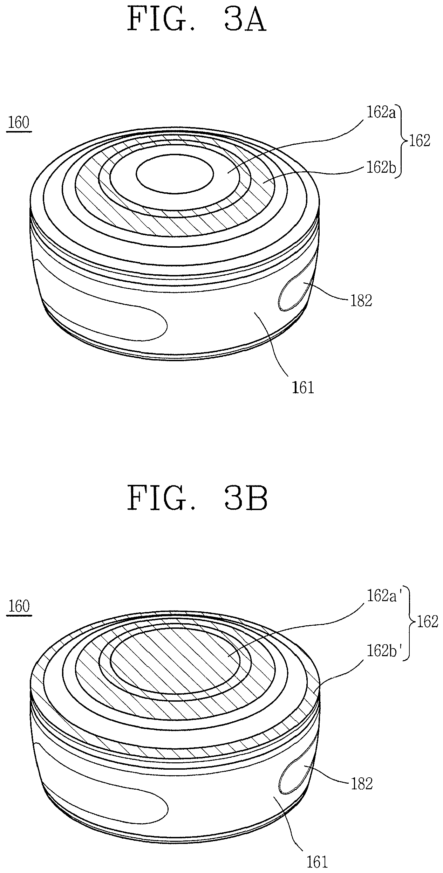

FIGS. 3A to 3C are views of body composition measuring devices according to further embodiments of the present invention when viewed from one direction. The body composition measuring device 160 of FIG. 3A includes a cylindrical body portion 161 having first and second sides, circular in shape, which face each other. An electrode portion is formed on both the first and second sides of the body portion 161. The electrode portion 162 includes first and second electrodes 162a and 162b, and the first and second electrodes 162a and 162b are spaced apart from each other and the second electrode 162b surrounds the first electrode 162a.

The first and second sides may have a curved surface that bulges further towards the center. This brings the electrode portion into better contact with the human body. In addition, a switch part 182 is provided on the side connecting the first and second sides of the body portion 161. The switch part 182 generates a control signal as it is pushed by a physical force from outside. Further, the control signal can control the power supply of the body composition measuring device 100, the execution of the body composition measurement function, etc.

The body composition measuring devices 100 according to these embodiments measure an impedance when a current is passed through the human body by bringing an area of the human body into the two electrode portions located on both sides of the body portion. Using additional information on the human body, as well as impedance measurements, the body composition (fat, fat-free mass, mineral, water, etc.) can be estimated. Hereinafter, a method of creating body composition data using the body composition measuring device 100 will be described.

Referring to FIG. 3B, a first electrode 162a' is provided at the center of a cross-section of the body portion 161, and a second electrode 162b' is provided along the edge of the cross-section. That is, the first and second electrodes are spaced apart from each other, and their shape and configuration are not limited so long as they both can come into contact with the user's body.

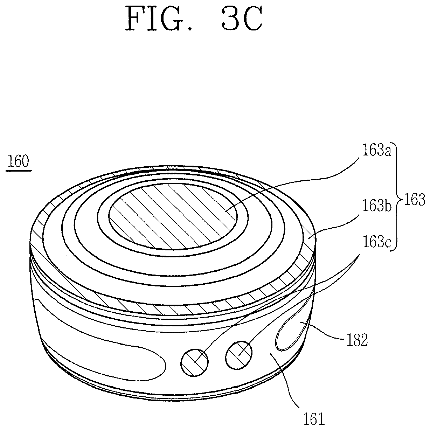

An electrode portion of the body composition measuring device 160 of FIG. 3C includes three electrodes. In particular, an electrode portion 163 includes first to third electrodes 163a, 163b, and 163c provided on a first side of the body portion 161. Further, the first and second electrodes 163a and 163b can be provided in substantially the same manner as the first and second electrodes of FIGS. 3A or 3B. In addition, the third electrode 163c is provided on the side connecting the first and second sides and includes a pair of electrodes placed in close proximity to each other.

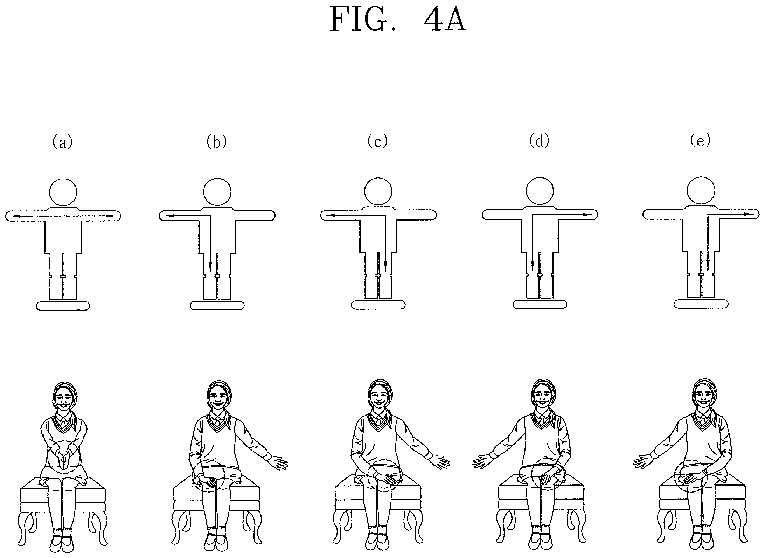

Next, FIG. 4A is a conceptual diagram explaining a method of estimating total body impedance using impedance measurements of different areas of the human body, obtained by the body composition measuring device 100 of the present invention. In particular, (a) of FIG. 4A illustrates a posture for measuring impedance from arm to arm. As shown, the user brings the first and second sides of the body portion into contact with both palms in order to measure impedance from arm to arm.

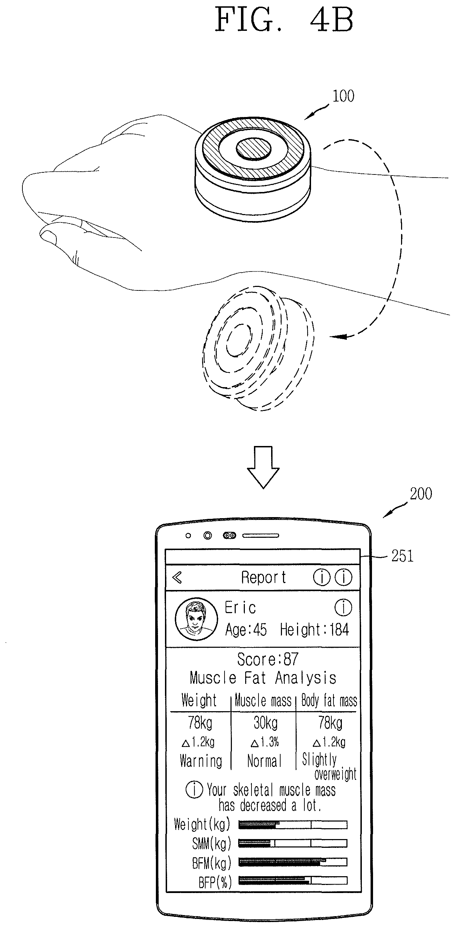

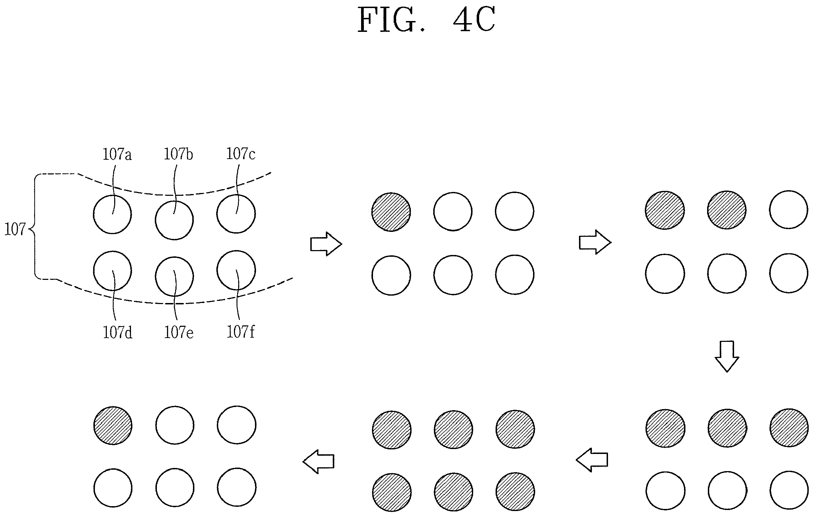

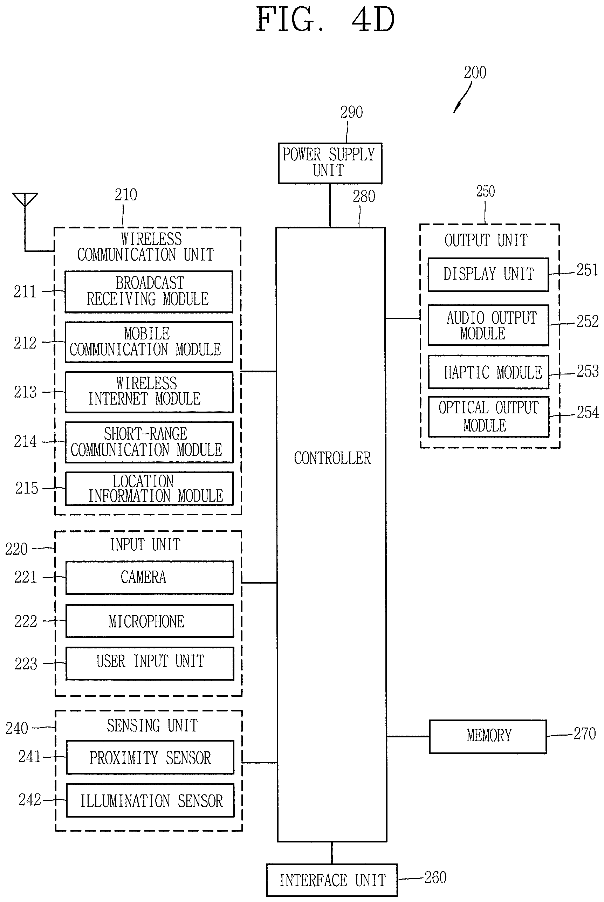

In addition, (b) of FIG. 4 illustrates a posture for measuring impedance from the right hand to the right leg. As shown, the user brings the first side of the body portion into contact with the right hand and the second side into contact with an area (e.g., knee) of the right leg, while sitting on the chair, in order to measure impedance from right hand to right leg. While the figure illustrates that the user is bringing the second side of the body portion into contact with the right knee, the second side of the body can be brought into contact with the right foot, instead of the right knee.

Also, (c) of FIG. 4 illustrates a posture for measuring impedance from the right hand to the left leg. As shown, the user brings the first side of the body portion into contact with the right hand and the second side into contact with a part of the left leg, while sitting on the chair, in order to measure impedance from right hand to left leg.

Next, (d) of FIG. 4 illustrates a posture for measuring impedance from the left hand to the right leg, and (e) of FIG. 4 illustrates a posture for measuring impedance from the left hand to the left leg, which are substantially identical to (c) of FIG. 4A and (b) of FIG. 4A, respectively.

In addition, the control part 101 or an external device's control part which receives impedance measurements can calculate body composition data by adding or subtracting the impedance measurements of different areas of the human body that are obtained in different postures. Further, the body composition measuring device 100 can measure only a part of the body the user wants. In this instance, desired data can be calculated by obtaining multiple impedance measurements in multiple postures and then subtracting the impedance measurement of a redundant area. For example, the composition of the belly can be estimated by measuring impedance from the left hand to the right leg, from the right hand to the left leg, from hand to hand, and from leg to leg.

When an impedance measurement is made in a particular posture, the control part 101 can determine which part of the human body the impedance measurement has been made of, by comparing the measurement data with stored reference data.

Next, FIG. 4B is a conceptual diagram explaining a control method for measuring the circumference/length of a part of the human body in order to create body composition measurement data. In particular, the body composition measuring device 100 according to this embodiment has a sensing part including a sensor for sensing rotation and movement of the body portion.

The sensing part can sense rotation when the body portion rotates in a particular direction and calculate the circumference when the body portion returns to the initial position. As shown in the drawing, the circumference of a wrist can be measured. In addition, the control part 101 can send circumference measurement data to the external device, and the circumference measurement data, along with an impedance measurement, can form body composition data.

FIG. 4C is a conceptual diagram explaining how the light-emitting regions in the output part emit light according to the measured level. In particular, the output part 107 may include first to sixth light-emitting elements 107a, 107b, 107c, 107d, 107e, and 107f. When the body composition measurement function is executed, the control part 101 can sequentially activate the light-emitting elements based on the measured level. For example, when the device is set to measure impedance in six postures, the light-emitting elements can be sequentially activated upon completion of the measurement of impedance in each posture.

When the device is set to measure impedance in three postures, and when the impedance measurements in the three postures are all obtained, all the light-emitting elements can produce light. That is, the control part 101 can control the output part based on a particular pattern so that the user is aware of the progression of the measurement function.

Next, FIG. 4D is a conceptual diagram explaining the components of an example of an external device 200 which can be connected wirelessly to the body composition measuring device 100 according to an embodiment of the present invention.

The mobile terminal 200 is shown having components such as a wireless communication unit 210, an input unit 220, a sensing unit 240, an output unit 250, an interface unit 260, a memory 270, a controller 280, and a power supply unit 290. Implementing all of the illustrated components is not a requirement, and that greater or fewer components may alternatively be implemented.

Referring to FIG. 4D, the mobile terminal 200 is shown having wireless communication unit 210 configured with several commonly implemented components. For instance, the wireless communication unit 210 typically includes one or more components which permit wireless communication between the mobile terminal 200 and a wireless communication system or network within which the mobile terminal is located.

The wireless communication unit 210 typically includes one or more modules which permit communications such as wireless communications between the mobile terminal 200 and a wireless communication system, communications between the mobile terminal 200 and another mobile terminal, communications between the mobile terminal 200 and an external server. Further, the wireless communication unit 210 typically includes one or more modules which connect the mobile terminal 200 to one or more networks. To facilitate such communications, the wireless communication unit 210 includes one or more of a broadcast receiving module 211, a mobile communication module 212, a wireless Internet module 213, a short-range communication module 214, and a location information module 215.

The input unit 220 includes a camera 221 for obtaining images or video, a microphone 222, which is one type of audio input device for inputting an audio signal, and a user input unit 223 (for example, a touch key, a push key, a mechanical key, a soft key, and the like) for allowing a user to input information. Data (for example, audio, video, image, and the like) is obtained by the input unit 220 and may be analyzed and processed by controller 280 according to device parameters, user commands, and combinations thereof.

The sensing unit 240 is typically implemented using one or more sensors configured to sense internal information of the mobile terminal, the surrounding environment of the mobile terminal, user information, and the like. For example, in FIG. 4D, the sensing unit 240 is shown having a proximity sensor 241 and an illumination sensor 242.

If desired, the sensing unit 240 may alternatively or additionally include other types of sensors or devices, such as a touch sensor, an acceleration sensor, a magnetic sensor, a G-sensor, a gyroscope sensor, a motion sensor, an RGB sensor, an infrared (IR) sensor, a finger scan sensor, a ultrasonic sensor, an optical sensor (for example, camera 221), a microphone 222, a battery gauge, an environment sensor (for example, a barometer, a hygrometer, a thermometer, a radiation detection sensor, a thermal sensor, and a gas sensor, among others), and a chemical sensor (for example, an electronic nose, a health care sensor, a biometric sensor, and the like), to name a few. The mobile terminal 200 may be configured to utilize information obtained from sensing unit 240, and in particular, information obtained from one or more sensors of the sensing unit 240, and combinations thereof.

The output unit 250 is typically configured to output various types of information, such as audio, video, tactile output, and the like. The output unit 250 is shown having a display unit 251, an audio output module 252, a haptic module 253, and an optical output module 254.

The display unit 251 may have an inter-layered structure or an integrated structure with a touch sensor in order to facilitate a touch screen. The touch screen may provide an output interface between the mobile terminal 200 and a user, as well as function as the user input unit 223 which provides an input interface between the mobile terminal 200 and the user.

The interface unit 260 serves as an interface with various types of external devices that can be coupled to the mobile terminal 200. The interface unit 260, for example, may include any of wired or wireless ports, external power supply ports, wired or wireless data ports, memory card ports, ports for connecting a device having an identification module, audio input/output (I/O) ports, video I/O ports, earphone ports, and the like. In some cases, the mobile terminal 200 may perform assorted control functions associated with a connected external device, in response to the external device being connected to the interface unit 260.

The memory 270 is typically implemented to store data to support various functions or features of the mobile terminal 200. For instance, the memory 270 may be configured to store application programs executed in the mobile terminal 200, data or instructions for operations of the mobile terminal 200, and the like. Some of these application programs may be downloaded from an external server via wireless communication. Other application programs may be installed within the mobile terminal 200 at time of manufacturing or shipping, which is typically the case for basic functions of the mobile terminal 200 (for example, receiving a call, placing a call, receiving a message, sending a message, and the like). It is common for application programs to be stored in the memory 270, installed in the mobile terminal 200, and executed by the controller 280 to perform an operation (or function) for the mobile terminal 200.

The controller 280 typically functions to control overall operation of the mobile terminal 200, in addition to the operations associated with the application programs. The controller 280 may provide or process information or functions appropriate for a user by processing signals, data, information and the like, which are input or output by the various components depicted in Fig. 4D, or activating application programs stored in the memory 270. As one example, the controller 280 controls some or all of the components illustrated in FIGS. 4D according to the execution of an application program that have been stored in the memory 270.

The power supply unit 290 can be configured to receive external power or provide internal power in order to supply appropriate power required for operating elements and components included in the mobile terminal 200. The power supply unit 290 may include a battery, and the battery may be configured to be embedded in the terminal body, or configured to be detachable from the terminal body.

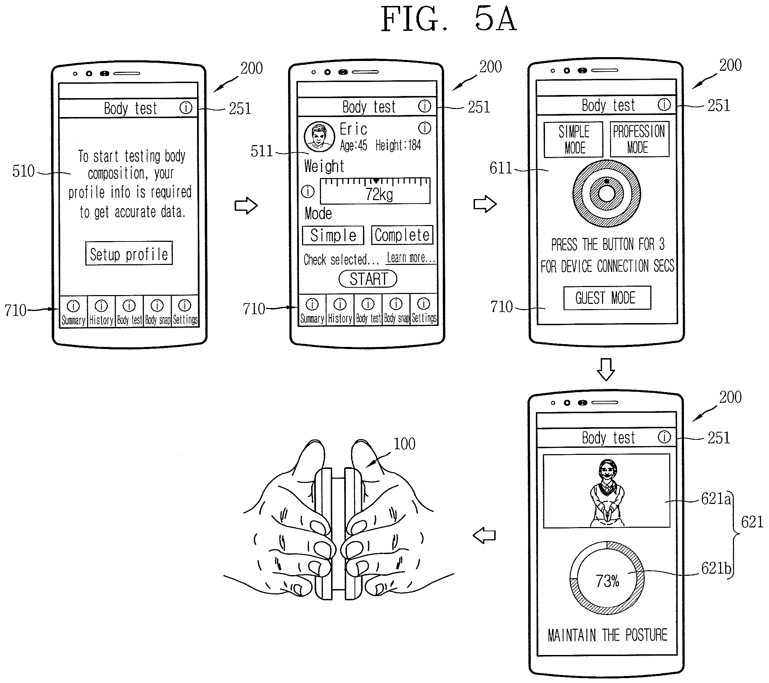

Next, FIGS. 5A to 5E are conceptual diagrams explaining a control method for a mobile terminal 200 that communicates wirelessly with the body composition measuring device 100 according to one embodiment. Referring to FIG. 5A, a method of controlling the operation of the body composition measuring device 100 in simple mode will be described.

In more detail, the body composition measuring device 100 and a mobile terminal 200 can be wirelessly connected. An application for operating the body composition measuring device 100 can also be executed on the display 251 when the mobile terminal 200 is wirelessly connected to the body composition measuring device 100. Alternatively, the application can be executed to establish a wireless connection with the body composition measuring device 100.

When the application is executed, the controller 280 of the mobile terminal 200 can control the wireless communication part 210 to send a start control signal for adjusting a zero point of the body composition measuring device. When the application is executed, an initial screen 510 appears on the display 251 of the mobile terminal 200. For example, a menu image 710 can appear along with the initial screen 510.

In more detail, the menu image 710 may include a plurality of icons for executing functions using data obtained by the body composition measuring device 100. For example, the menu image 710 may include a summary icon that shows user information or guidance information, a history icon that shows various information from stored measurement data, a body test icon for measuring body composition data, a body snap icon that shows images which are stored (or to be stored) along with the body composition data, and a setting icon for setting the operation of the body composition measuring device.

The initial screen 510 may include text or/and images that include guidance information for operating the body composition measuring device 100. Further, the initial screen 510 can display a graphic image for entering information on the user of the body composition measuring device 100.

User information can be entered based on touch input on the graphic image. The user information, as used herein, includes information on the user of the mobile terminal 200 connected to the body composition measuring device 100. For example, the user information can include the user's name, photograph, weight, height, and gender. In addition, if the user information includes information regarding past surgeries, the controller 280 can output guidance information telling the user to stop using the body composition measuring device.

Also, the display 251 of the mobile terminal 200 displays first screen information 511 before collecting body composition data by the body composition measuring device 100. As shown, the first screen information 511 can be output along with the menu image 710, and other functions can be executed based on a touch on the menu image 710. The first screen information 511 may include a user profile and a graphic image START for receiving touch input before starting body composition measurement using the body composition measuring device 100.

Based on a touch input on the graphic image, the controller 280 of the mobile terminal 200 controls the display 251 to display a first execution screen 611 for operating the body composition measuring device 100. Further, the first execution screen 611 may include graphic images for controlling the measurement mode of the body composition measuring device 100. The graphic images may correspond to a simple mode, a complete mode, and a guest mode, respectively. Moreover, the first execution screen 611 may include guidance information for wireless connection with the body composition measuring device 100. The guidance information may include text or/and images.

When wirelessly connected to the body composition measuring device 100, the controller 280 of the mobile terminal 200 implements the body composition measuring device 100 in a particular selected mode. Further, the mobile terminal 200 transmits a wireless signal corresponding to the selected mode to the body composition measuring device 100.

For example, when the body composition measuring device 100 is operated in a simple mode, the display outputs a first measurement screen 621 including measurement guidance information 621a and measurement status information 621b. The measurement guidance information 621a includes an image, etc. that represents a posture the user has to make in the selected mode.

Also, the measurement status information 621b includes an image that represents the amount of work completed for a measurement being made by the body position measuring device 100, and the image may change as the measurement goes on. Thus, the user can check progress on the measurement and maintain the correct posture during execution of the body composition measurement function. In the simple mode, the user has to maintain the posture until completion of the body composition measurement. That is, in simple mode, the body composition of a part of the human body is between two fixed areas of the human body.

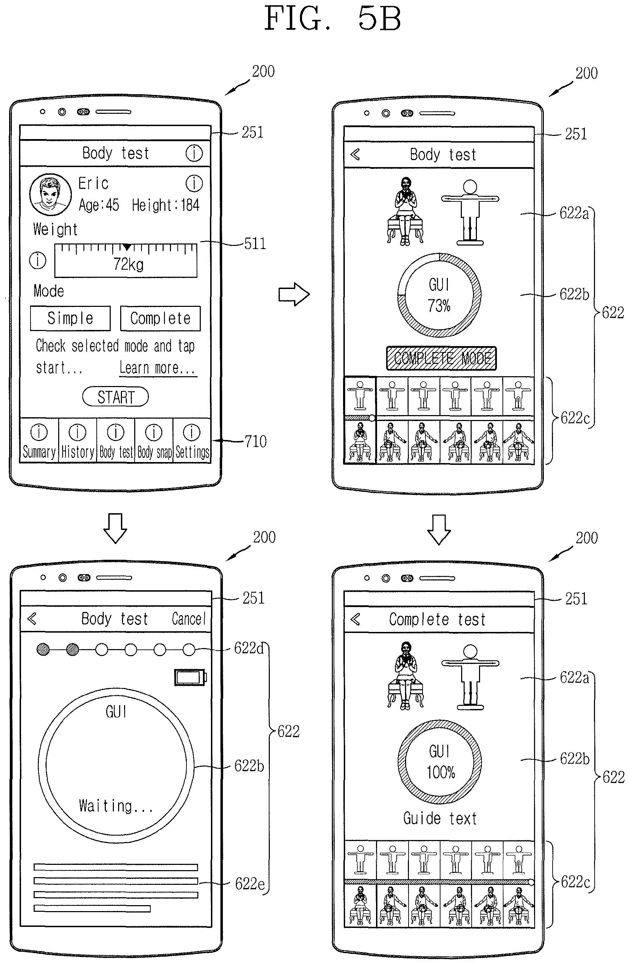

Referring to FIG. 5B, a measurement screen is shown which appears on the mobile terminal 200 when the body composition measurement function is performed in a complete mode. In the complete mode, the user makes multiple postures to obtain impedance measurements of multiple parts of the human body, and then total body composition data is calculated. Accordingly, the user brings the body composition measuring device 100 into contact with the body in various postures, in order to obtain impedance measurements of different parts of the body.

When the body composition measurement function is executed by the body composition measuring device 100, the first screen information 511 appears on a second measurement screen 622. The second measurement screen 622 includes posture guidance information 622a for measuring a target region and first measurement status information 622b and second measurement status information 622c that represent the body measurement status.

Further, the body composition measuring device 100 according to this embodiment performs measurement for a specific number of postures in order to measure an area of the human body preset by the user. For example, to analyze total body composition, the user can be guided to make a measurement in six postures. The posture guidance information 622a may include images of the user's posture for making a measurement while holding the body composition measuring device 100.

The first measurement status information 622b may include a graphic image and text that represent the amount of work remaining and the amount of work completed. The graphic image of the measurement status gradually changes with time.

The second measurement status information 622c includes a plurality of images that are arranged in one direction and represent postures for measuring different body regions. In addition, the user sequentially makes the postures displayed on the images and measures the impedance of the body regions. Also, the second measurement status information 622c includes a progress bar that extends along the images arranged in a line as impedance measurements are obtained. When the impedance of each part of the human body is obtained and transmitted by the body composition measuring device 100 (or a wireless signal indicating that the impedance measurement of each region has been made), the controller 280 of the mobile terminal 200 extends the progress bar to indicate that the result of measurement of that region has been obtained.

In addition, the second measurement screen 622 includes the second measurement status information 622c, third measurement status information 622d indicating a measured status, and guide information 622e. Further, the third measurement status information 622d is implemented in the form of a graphic image indicating steps, and the graphic image includes images corresponding to measurement steps. Each of the images is transformed when each measurement is completed. Accordingly, a user can check a measurement completed status through a transformation of the images. The controller 280 can also be transformed into another shape or color, according to a good or poor measurement state.

Also, the guide information 622e may be implemented in the form of text. For example, the guide information 622e can be formed as a text indicating each measurement posture, and include an explanation for guiding a posture change according to a measured status. Thus, a user can check a plurality of measurement postures through texts and/or images. According to this embodiment, the user can learn which posture to make to measure each area of the body and find out whether the body's impedance measurement has been obtained with each posture.

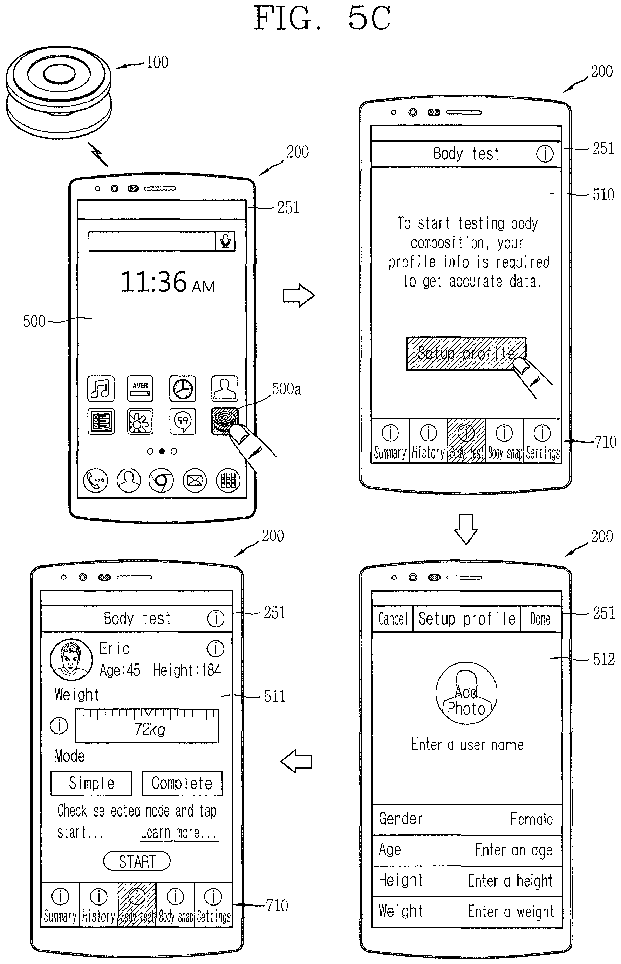

Referring to FIG. 5C, a control method of setting up information to operate the body composition measuring device 100 by the mobile terminal 200 will be described. As shown, the display 251 of the mobile terminal 200 can display an icon 500a for an application that is wirelessly connected to the body composition measuring device 100 to control the operation of the body composition measuring device 100 and that performs wireless communication to receive collected impedance measurements or body composition measurements.

Further, the controller 280 of the mobile terminal 200 displays an information input screen 510 based on touch input on the icon 500a. If this is not the first time the application is executed, or specific information is already entered by the user, the stored information can be output.

In addition, the information input screen 510 can be output along with the menu image 710 for executing other functions of the application. As shown, the display 251 displays an input window 512 based on touch input on the information input screen 510. In particular, the input window 512 includes an input part for entering the user's basic information and body information. The controller 280 can store the profiles of multiple users in the memory of the mobile terminal 200, and analyze and store impedance data measured by the body composition measuring device 100, along with the user profiles.

The display 251 stores a user profile before an impedance measurement is measured by the body composition measuring device 100. Further, the first screen information 511 may include the user's photo, name, gender, height, and age. The first screen information 511 may also include a graphic image that receives touch input to form a control signal for measuring the user's body composition.

At least part of the user's profile stored on the mobile terminal 200 can also be transmitted to the body composition measuring device 100. In this instance, the body composition measuring device 100 can calculate body composition measurement data by using the received user profile. According to this embodiment, the user can get more accurate measurement data from the body composition measuring device 100 by entering additional information about the body.

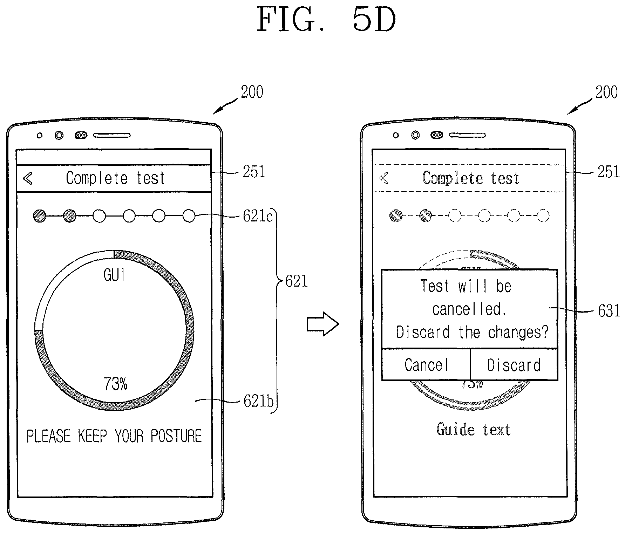

Referring to FIG. 5D, a control method for providing information about a measurement error will be described. In particular, the controller 280 of the mobile terminal 200 controls the display 251 to display a first measurement screen 621 that represents the progress of a measurement being made by the body composition measuring device 100.

The first measurement screen 621 includes a measurement status image 621c including as many images as the postures for body measurement. When a measurement in a position is completed, the measurement status image 621c changes to indicate the completion of the measurement in that position. The first measurement screen 621 includes measurement status information 621b. The measurement status information 621b may be composed of a graphic image and/or text that shows the percentage completed.

Upon detecting an abnormality such as when impedance is out of a set normal range during estimation while in contact with the human body, the body composition measuring device 100 can send an error signal to the mobile terminal 200. This abnormality includes when the impedance changes abruptly or is out of the normal range due to a change in the user's posture or a sudden current variation.

Upon receiving the error signal, the controller 280 of the mobile terminal 200 controls the display 251 to display an error window 631 showing this error signal. The error window 631 includes text that shows the types of measurement functions of the body composition measuring device 100 and checks whether a measurement result containing abnormality information has been deleted or not.

According to this embodiment, if the user changes their posture abruptly, they are informed about this in the form of abnormality information, rather than from a measurement result, which allows for more accurate body measurement results.

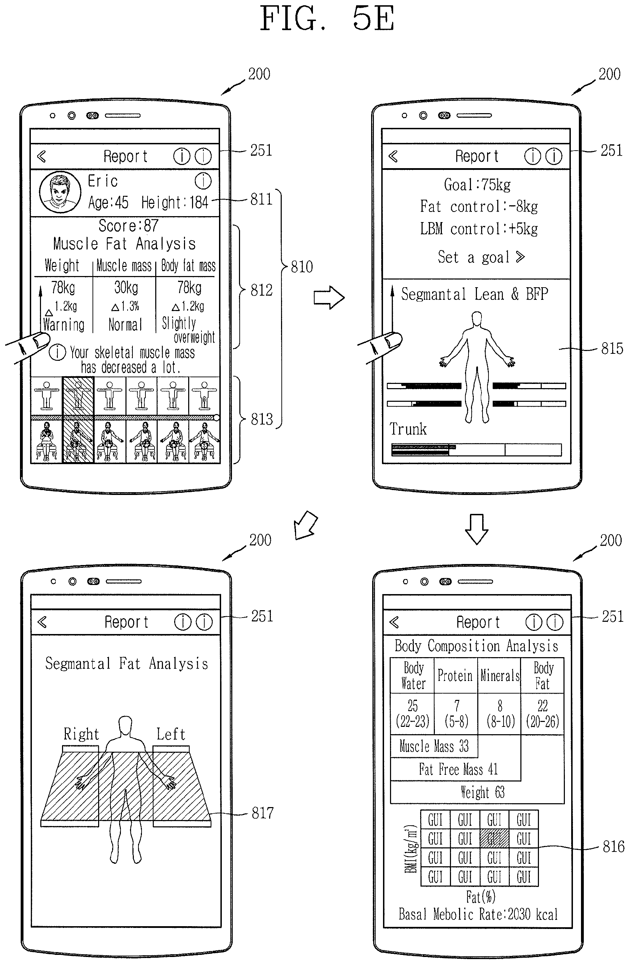

Referring to FIG. 5E, when the measurement of all the set postures is completed, the body composition measuring device 100 transmits the measurement result to the mobile terminal 200 with which it is in wireless communication. Measurement data is created from the measurement result transmitted to the mobile terminal 200.

The mobile terminal 200 creates body composition data using the impedance measurement made by the body composition measuring device 100, and includes a first result screen 810 including the body composition measurement data. The first result screen 810 includes the user's basic information 811, a measurement result table 812, and a measurement status image 813. The measurement result table 812 may include weight, muscle mass, body fat mass, and a guidance message ("Your skeletal muscle mass has decreased a lot.") which gives a comparison with past measurement results, and may include text, a comparison graph, etc.

The measurement status image 813 includes a plurality of posture images for measurement, and the images may change to indicate the quality of measurement in each posture. If, in a particular posture, too many movements are detected during measurement or error information is included, the display 251 changes the corresponding image. Accordingly, the user can predict that the impedance of a particular body region may be inaccurate, and if necessary, perform a re-measurement.

For example, the plurality of posture images can be arranged in the order of measurement. If the quality of measurement is low, the posture images can be dimmed. Further, the controller 280 controls the display unit 251 to output analysis information, based on a specific touch input applied to the first result screen 810. First analysis information 815 may include images indicating measurement results on parts of a user's body. In addition, he controller 280 can set a target value with respect to each part of the user's body, based on a touch input applied to each of the images.

Second analysis information 816 includes a plurality of images corresponding to a plurality of body type information distinguished from each other according to a specific reference. Also, an image corresponding to measured data, among the plurality of images, is displayed in another color or shape. Thus, a user can check his or her body type information in the form of images.

Third analysis information 817 is implemented in the form of an image indicating a balance of a human body. The analysis information 817 includes a graphic image indicating a balanced status of right and left parts of a human body, formed based on measured data. The graphic image may be formed to overlap an image indicating a human body, and may be represented in a different shape based on data.

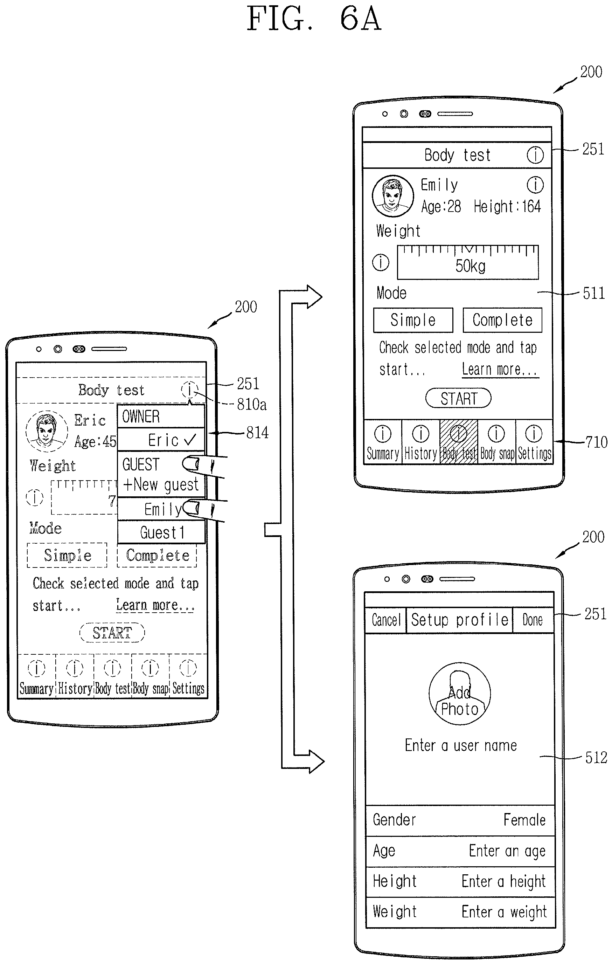

Next, FIGS. 6A to 6C are conceptual diagrams explaining the mobile terminal 200 performing the measurement function and a control method for the body composition measuring device 100. Referring to FIG. 6A, when the mobile terminal 200 is wirelessly connected to the body composition measuring device 100, and a particular application is executed, pre-stored information about a particular user and a graphic image for executing the body composition measuring device 100's function can be displayed. The graphic image includes a select icon 810a for selecting more users.

The controller 280 of the mobile terminal 200 outputs a select Menu window 814 based on touch input on the select icon 810a. The select Menu window 814 includes saved users and the user who recently had their body composition measured. When another user in the select Menu window 814 is selected, the controller 280 of the mobile terminal 200 controls the display 251 to display first screen information 511 again, including information on the selected user.

A graphic image START can also be displayed based on touch input on the first screen information 511 of the selected user. The controller 280 can receive touch input on the graphic image START and send a control signal for measuring the user's body composition.

Further, the controller 280 can save new users based on a touch input on the select Menu window 814. When New Guest is selected from the select Menu window 814, the display 251 can be controlled to display the input window 512 for entering new information. Accordingly, the body compositions of multiple users can be measured by means of the body composition measuring device 100 connected to the mobile terminal 200.

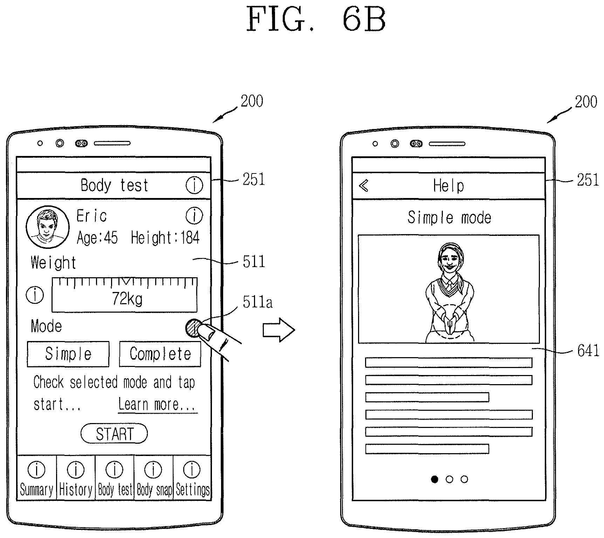

Referring to FIG. 6B, a method of providing guidance information will be described. As shown, a first guidance screen 641 appears on the first screen information 511 based on touch input on a guidance icon 511a. The first guidance screen 641 includes text and/or image illustrating how to measure body composition when the body composition measuring device 100 is in simple mode. The image describes a single posture, and may be in the form of video.

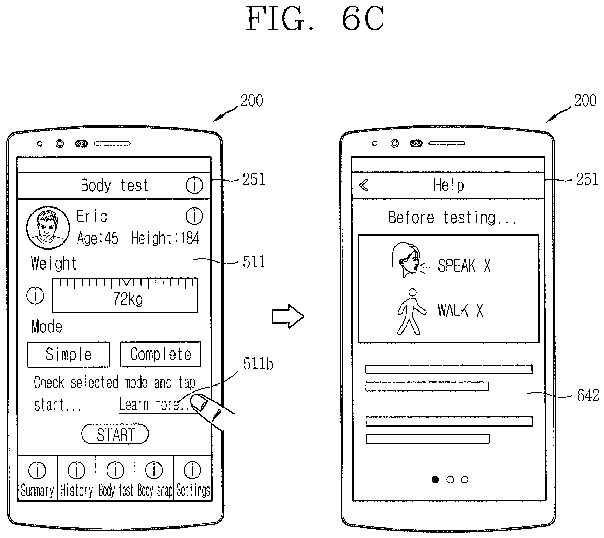

Referring to FIG. 6C, the display 251 displays a second guidance screen 642 based on touch input on guidance text 511b included in the first screen information 511. The first screen information 511 includes text for briefly describing how to do measurement. The guidance text 511b includes, but is not limited to, text information which tells the user to select if more information is needed.

The second guidance screen may include detailed descriptions of how to do measurement in the complete mode as well as in the simple mode and of things to keep in mind, a description of the body composition measuring device 100, and a method of measurement result estimation. According to the above embodiments, the user can enter new users on the first screen information or be provided with guidance information.

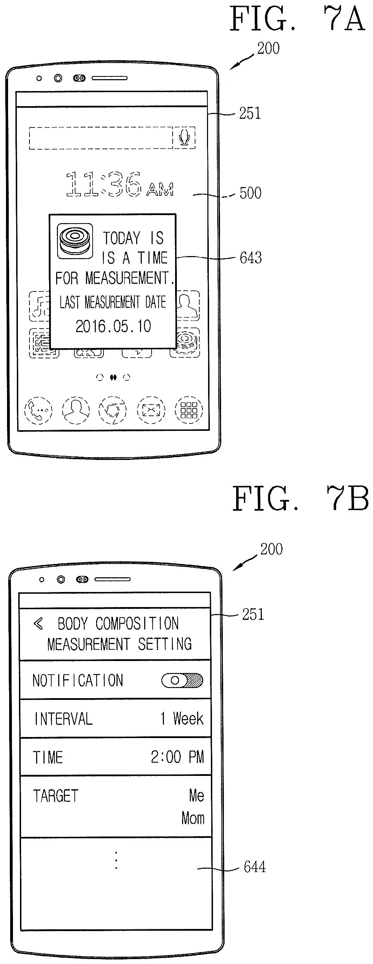

Next, FIGS. 7A and 7B are conceptual diagrams explaining a control method for issuing a notification of measurement from the body composition measuring device 100. Referring to FIG. 7A, the controller 280 of the mobile terminal 200 outputs notification information 643 for executing the body composition measurement function at a specified time. The notification information 643 may be visual information that is displayed on the display 251. However, the notification information 643 is not limited to this and may include voice information (audio data), vibration, etc.

For example, the notification information 643 may include information regarding the last date of measurement. In more detail, the controller 280 can establish a wireless connection with the body composition measuring device 100 based on touch input on the notification information 643 and execute a corresponding application

The notification information 643 may appear any time during use of the mobile terminal 200. For example, the notification information 643 may appear as a pop-up window while a home screen page 500 is being output. Further, the controller 280 can allow the mobile terminal 200 to display notification information from the body composition measuring device 100 at a set time.

Specifically, the controller 280 can perform wireless communication with the body composition measuring device at the alarm time and send a wireless signal to the body composition measuring device 100 to issue a notification. The body composition measuring device 100, upon receiving the wireless signal, can operate to measure body composition.

Referring to FIG. 7B, the user can set the alarm to issue alarm information by using the mobile terminal 200. For example, the display 251 of the mobile terminal 200 can display a setting screen 644 illustrating whether the alarm is enabled or not, the alarm interval, the alarm time, and a list to select a target user from.

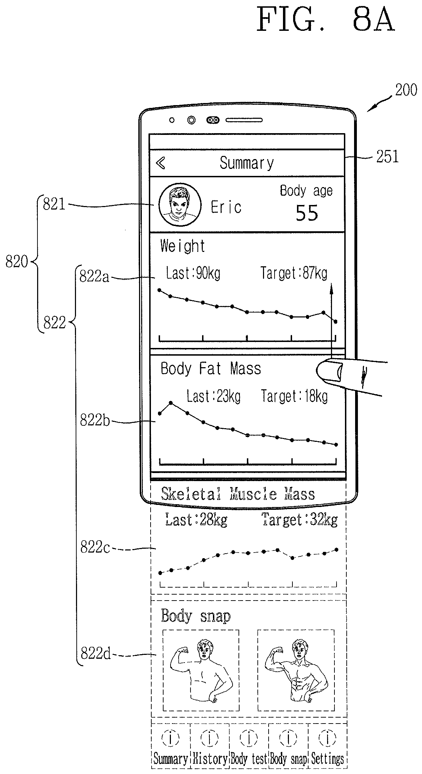

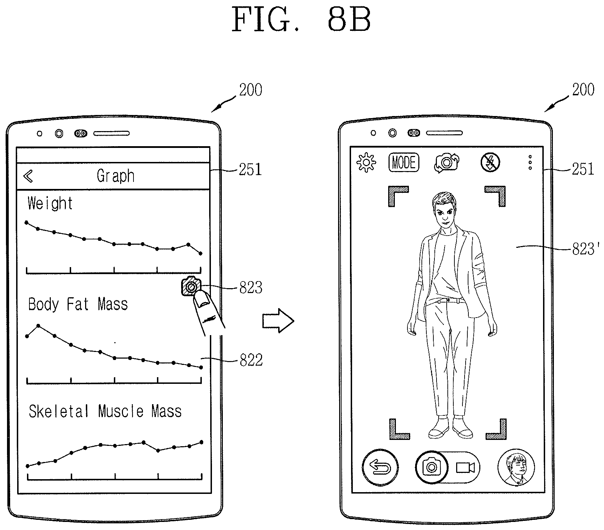

Next, FIGS. 8A and 8B are conceptual diagrams explaining a control method for providing a measurement result obtained by the body composition measuring device 100. Referring to FIG. 8A, the controller 280 controls the display 251 to display a second measurement result screen 820 using measurement data received from the body composition measuring device 100.

The second measurement result screen 820 includes a user profile 821 corresponding to a measurement result and a plurality of result pages 822. The result pages are not limited in number, but as shown in FIG. 8A, can include first to fourth result pages 822a, 822b, 822c, and 822d. The first to fourth result pages 822a, 822b, 822c, and 822d can contain results in different categories and show measurement results analyzed in text, images, graphs, etc.

The first result page 822a represents a change in weight (which can be calculated using stored information), the second result page 822b represents a change in body fat mass, and the third result page 822c may represent a change in skeletal muscle mass. The fourth result page 822d can include images that were captured on the date of measurement. It is preferable that the captured images are stored on the mobile terminal 200 during body composition measurement by the body composition measuring device 100, but the present invention is not limited to this. Further, the images are selected by the user, and may be the ones that were captured around the date of measurement.

The first to fourth result pages 822a, 822b, 822c, and 822d are sequentially arranged, and selectively output to the display 251. For example, the first to fourth result pages 822a, 822b, 822c, and 822d can be sequentially output based on a touch on the display 251. The order in which the plurality of result pages are output can also be changed as the user likes, and the each result page may include a target value that is set by the user or proposed for health.

Referring to FIG. 8B, the result graph screen 822 includes a capture icon 823 for capturing images. In addition, the controller 280 enables the camera 221 of the mobile terminal 200 based on touch input on the capture icon 823. Then, an image 823' captured by the camera 221 is output on the display 251. The controller 280 of the mobile terminal 200 can also store the image captured by the camera 221, along with the result data.

In more detail, a captured image can be stored along with result data for a particular date. According to this embodiment, the user can store an image of their body along with a result of body composition measurement and compare them. Since an image can be captured immediately while an application working in conjunction with the body composition measuring device 100 is enabled, this saves the user the hassle of having to go through multiple steps.

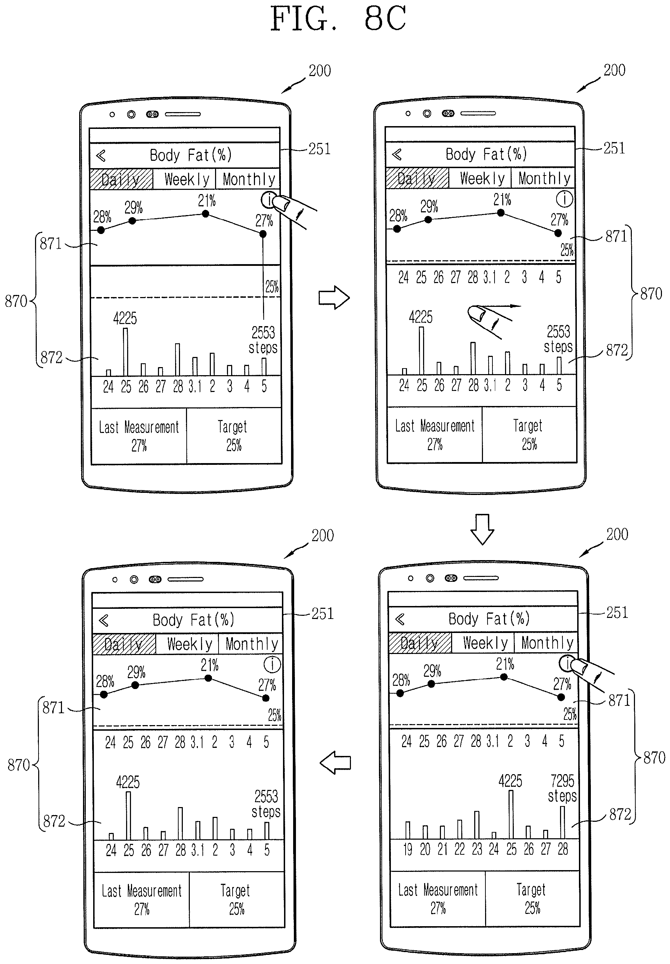

Next, FIG. 8C is a conceptual view illustrating a measurement result screen which displays a body fat ratio (percentage) and the number of steps. As shown, a seventh measurement result screen 870 according to this embodiment includes a first graph 871 having data on a change of a body fat ratio according to a time lapse, and a second graph 872 having data on a change of the number of steps according to a time lapse. For instance, the body fat ratio and the number of steps may include data measured by days of the week. The number of steps can also be measured by a sensing unit mounted to the mobile terminal 100 and configured to sense a movement.

The first and second graphs 871, 872 are displayed such that data on the body fat ratio and the number of steps, measured on the same time, is output together. In addition, data measured on the same date is displayed in a vertical direction of the mobile terminal 100. Based on a touch input applied to the second graph 872, the controller 280 controls the display unit 151 to display data on different dates. In this instance, data on the number of steps and the body fat ratio can be output in a vertical direction. Here, the first graph 871 is not changed.

If a touch input is applied to the graphic image included in the seventh measurement result screen 870, the controller 280 controls the display unit 151 to output the data on the number of steps and the body fat ratio measured on the previous dates, in an aligned manner. Thus, a user can be provided with data on the body fat ratio, together with data on the number of steps.

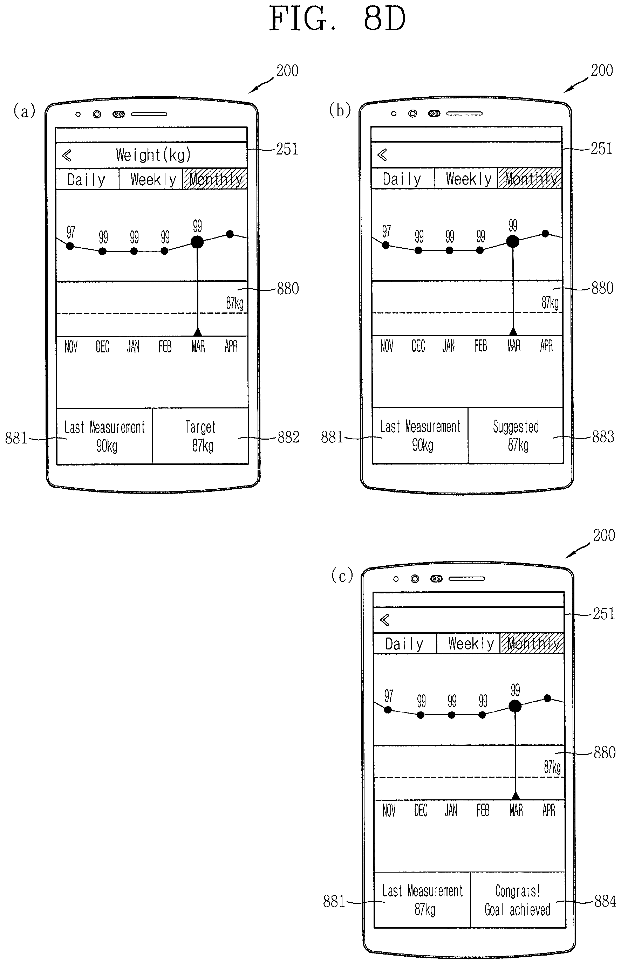

FIG. 8D is a conceptual view illustrating an eighth measurement result screen indicating a change of a weight. Referring to FIG. 8D, the eighth measurement result screen 880 includes data on a change of weights measured according to a time lapse. Also, the eighth measurement result screen 880 includes information on a target weight preset by a user.

Referring to FIG. 8D(a), the eighth measurement result screen 880 may include a first region 881 having data on a most-recently measured weight, and a second region 882 having information on a target weight set by a user. In addition, when a touch input is applied to the second region 882, a target value setting screen to change the target weight can be output.

Referring to FIG. 8D(b), if a user has not set a target weight, the controller 280 displays a third region 883 indicating a suggested weight, without displaying the second region 882. The suggested weight is an analysis result through a change of a user's body, etc. Referring to FIG. 8D(c), if the target weight set by the user is achieved, the controller 280 can display, on the display unit 151, a fourth region 884 including a message indicating that the user has succeeded in achieving the target value. Accordingly, the user can set a target weight, and may check whether the target weight has been achieved or not together with a change of weights.

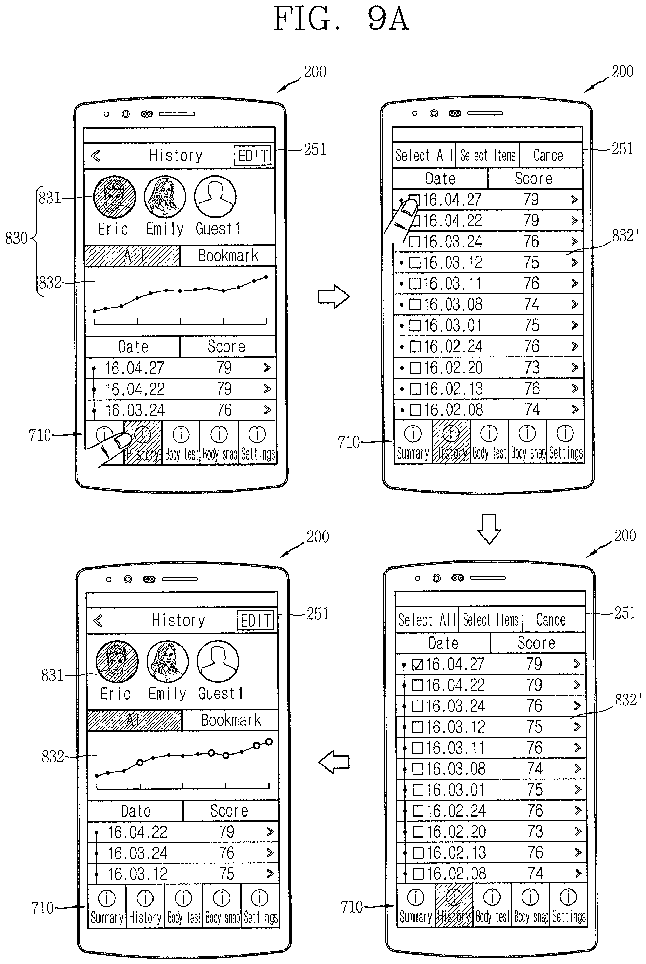

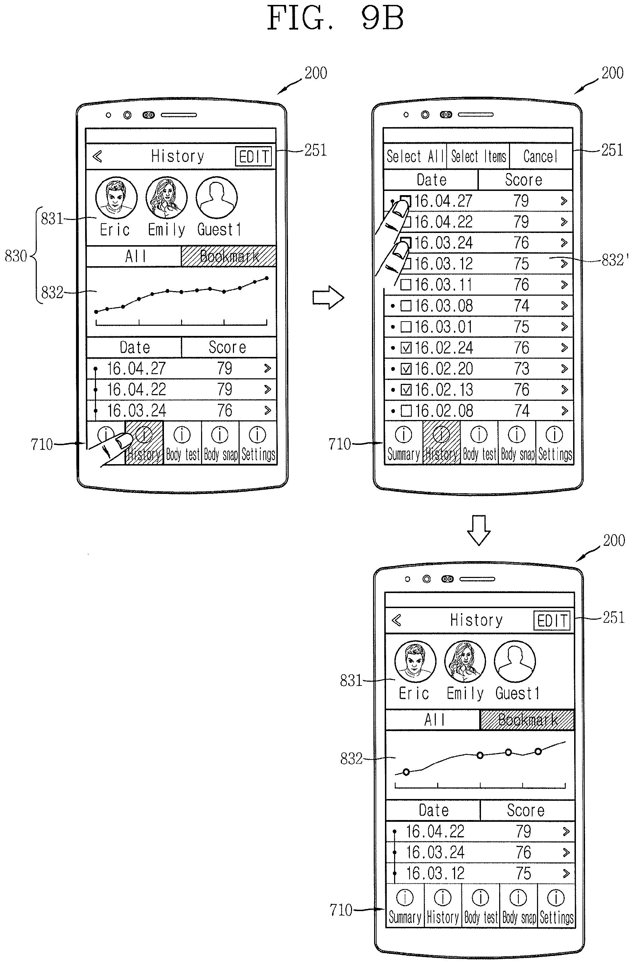

Next, FIGS. 9A and 9B are conceptual diagrams explaining a method of controlling result data according to one embodiment of the present invention. Referring to FIG. 9A, a third measurement result screen 830 is output on the display 251 of the mobile terminal 200. The third measurement result screen 830 provides result data which is measured by the body composition measuring device 100 and stored in the memory. The third measurement result screen 830 includes user profiles 831 of multiple users and a result page 832 for a selected user. The result page 832 may include a graph of body composition results for a particular period of time and a list of measurement data.

When the History menu is selected from the menu image 710, a list screen 832' of measurement data sorted by date. The list screen 832' may include items sorted by date measured, and the user can select data for a particular date. The controller 280 of the mobile terminal 200 can change the graph to display data the user selects from the list screen 832'. Further, the graph may include points indicating measurement results for dates of measurement, and the points corresponding to the data selected by the user can be highlighted. Accordingly, the user can output the selected data to make it noticeable.

Referring to FIG. 9B, the display 251 of the mobile terminal 200 can only display bookmarked result data. In more detail, the controller 280 can designate some of the result data based on the user's touch input on the list screen 832'. If the user selects to only show bookmarked data, the controller 280 outputs a graph consisting only of result data selected from the list screen 832'.

According to this embodiment, the memory 270 of the mobile terminal 200 can store result data created by the body composition measuring device 100, and only some of the stored result data can be output according to the user's setting. Also, the user can check for changes in their body on a per-date basis, especially because a graph consisting only of selected result data may be created.

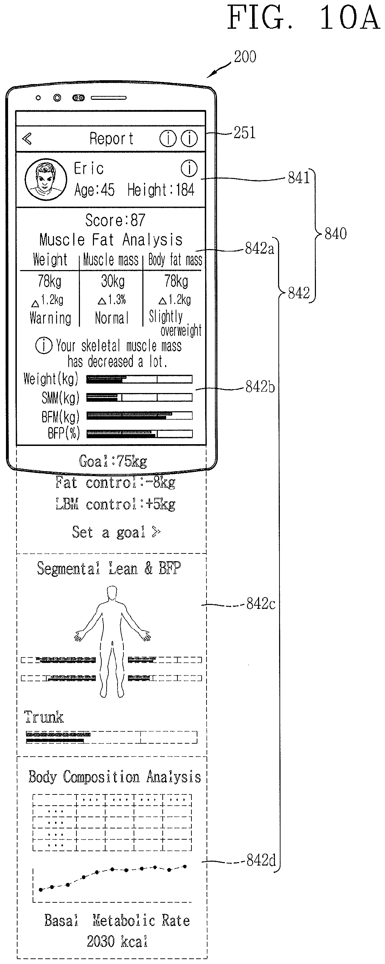

Next, FIGS. 10A to 10D are conceptual diagrams explaining a control method for creating target data using a measurement result screen. In particular, FIG. 10A illustrates a fourth measurement result screen 840 according to one embodiment. The fourth measurement result screen 840 includes a user profile 841 and a plurality of result pages 842. In more detail, the user profile 841 is information about a user who had their body composition measured by the body composition measuring device 100, and may include an image, name, age, height, etc. The controller 280 can also select another user based on touch input on the user profile 841.

Further, the plurality of result pages 842 are sequentially arranged and selectively displayed on the display 251. The controller 280 can sequentially output the plurality of result pages 842 based on a particular type of touch input (e.g., drag) on the display 251. The plurality of result pages 842 may include body composition data obtained by analyzing the measurement data. For example, the plurality of result pages 842 may include first to fourth result pages 842a, 842b, 842c, and 842d. In addition, the first result page may include numerical values representing the weight, skeletal muscle mass, and body fat mass and the corresponding analysis data (warning, normal, slightly overweight).

The second result page 842b includes graphs with the above numerical values. Graphs presenting discrete data are arranged to provide a relative comparison of the numerical values. The third result page 842c shows analysis data for each area of the human body. The fourth result page 842d may include an analysis graph showing an estimated basal metabolic rate.

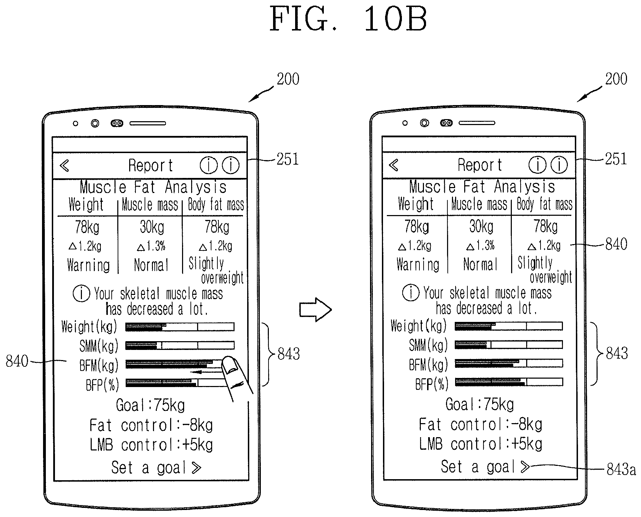

Referring to FIG. 10B, the controller 280 sets target data based on touch input on the fourth measurement result screen 840. For example, the fourth measurement result screen 840 may include a control image 843 that is in the form of graphs with numerical values that receives touch input. The control image 843 may include graphs representing the numerical values of different categories. The numerical value in a graph may be changed based on a touch on the graph. For example, the touch may involve dragging the graph from one end to the other.

Based on the touch, the controller 280 can change the shape of the graph and therefore change the numerical value. When a touch is applied onto the control image 843, the controller 280 can activate a setting image 843a. After the body composition values are changed using the control image 843, the changed values are set as target data based on a touch on the setting image 843a. That is, the user can see their body composition data obtained by the body composition measuring device 100 immediately through a result data screen, and intuitively set target data by changing the result data screen.

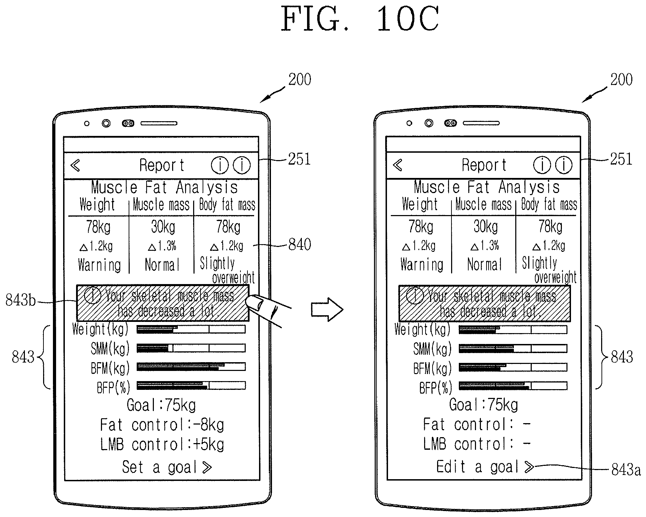

Referring to FIG. 10C, the fourth result data screen 840 can include a control image 843 and analysis data 843b. In more detail, the analysis data 843b can include text and/or images which describe any negative condition of the human body or any negative change in the human body by analyzing stored result data and the recent measurement data or comparing recommended values and the recent measurement data.

A touch input can be applied on the analysis data 843b, and the controller 280 can set target data based on the touch on the analysis data 843b. Further, the controller 280 sets stored recommendation data as target data based on the touch input on the analysis data 843b. The controller 280 can also change the control image 843 based on the target data setting.

After the control image 843 is changed based on the target data setting, the controller 280 can set and store the target data based on touch input on the setting image 843a. According to this embodiment, the user can set target data by using recommendation data suitable for the user, without having to set target data for themselves.

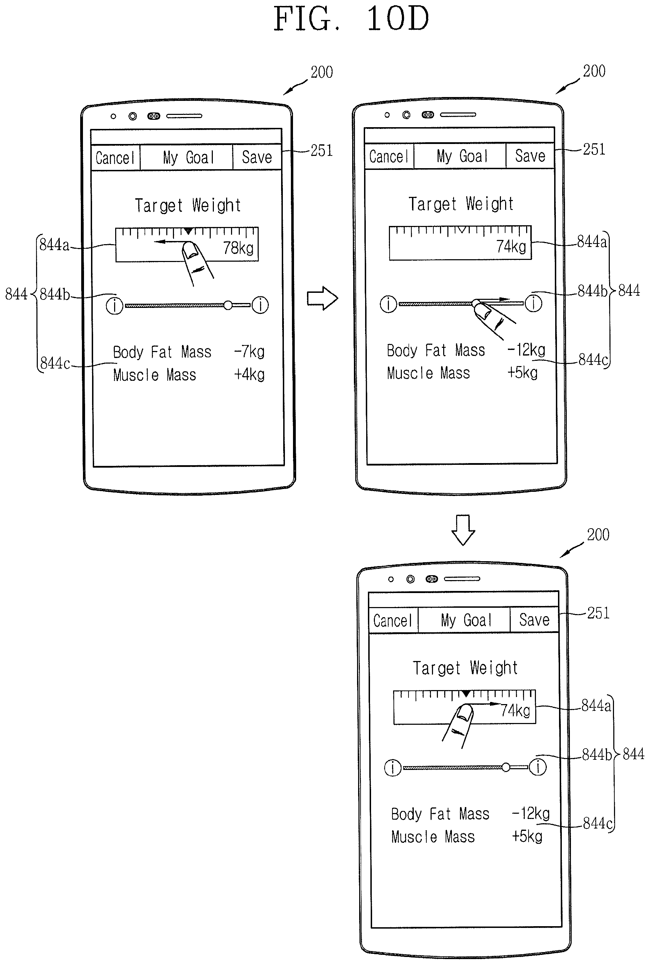

Referring to FIG. 10D, a target setting screen 844 for setting target data will be described. In particular, the display 251 includes first and second control images 844a and 844b and numerical data 844c. The first control image 844a represents current body composition data (e.g., weight). The first control image 844a also represents the type of body composition data and numerical values. When the type of body composition data is changed based on a touch on the first control image 844a, the second control image 844b is changed too.

The controller 280 sets target data based on touch input on either of the first and second control images 844a and 844b. That is, the second control image 844b is changed based on touch input on the first control image 844a, and the first control image 844a is changed based on touch input on the second control image 844b. Also, the numerical data varies with target data that is set based on a touch on either the first or second control image 844a or 844b. That is, the user can set a target based on either the first or second image 844a or 844b and see changes in numerical values through the numerical data 844c.

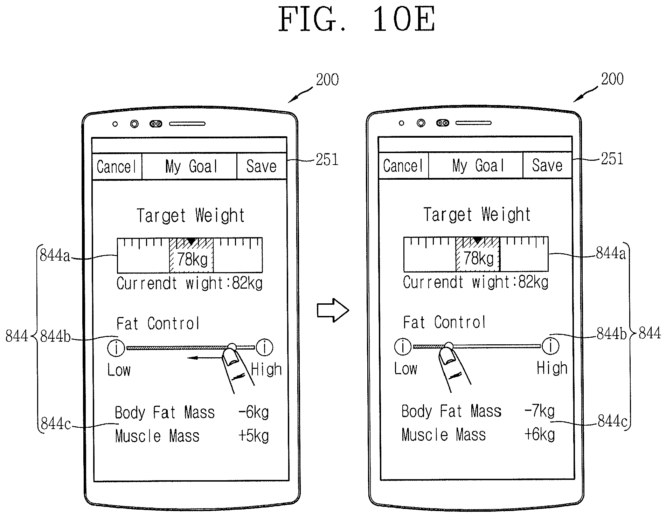

Next, FIG. 10E is a conceptual view illustrating a target setting screen which explains a target value. Referring to FIG. 10E, the display unit 251 displays first and second control images 844a, 844b, and numeric information 844c. The first control image 844a indicates data on a user's current weight, and the second control image 844b indicates a control image for setting a target weight.

The target weight may be set into a low fat type and a high fat type, by applying a dragging touch input to the second control image 844b. In this instance, the target weight is not changed. That is, the target weight may be set by controlling a fat mass and a muscle mass, in a state where the weight value is not changed.

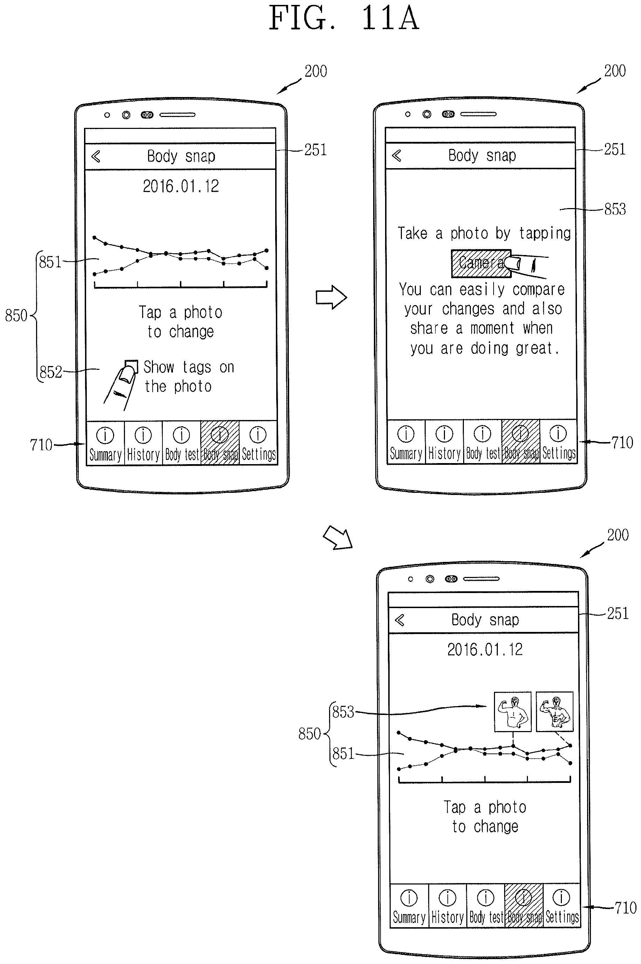

FIGS. 11A to 11D are conceptual diagrams explaining a control method for storing photographic data along with result data according to one embodiment. Referring to FIG. 11A, the display 251 displays a fifth measurement result screen 850. The fifth measurement result screen 850 may include a graph representing created body composition result data. However, the type of visual data 851 representing the body composition result data is not limited to graphs.

The fifth measurement result screen 850 may be output along with a menu image 710 for executing a particular function. The controller 280 can allow the user to select a body snap function that is included in the menu image 710 and that adds/edits photographic data. When the body snap function is selected, the controller 280 can output control content ("Show tags on the photo.") 852 on the fifth measurement result screen 850 to show photo tags. The controller 280 can output a guidance screen 853 for photo tagging based on touch input on the control content 852. The outputting of the guidance screen 853 may be omitted.

The controller 280 tags stored photographic data on the result data included in the visual data 851. For example, if the visual data 851 is composed of a graph 851 with a plurality of points, at least one photo 853 is displayed adjacent to a particular point. The memory 270 tags and stores a particular photograph at a point corresponding to measurement result data obtained on a particular date, based on a control command from the user.

The particular photo may be, but not limited to, a photo that was captured when the body composition measuring device 100 was operating. Accordingly, the user can see changes in their body more accurately through photos associated with measurement data, along with the visual data 851 which is in the form of a graph.

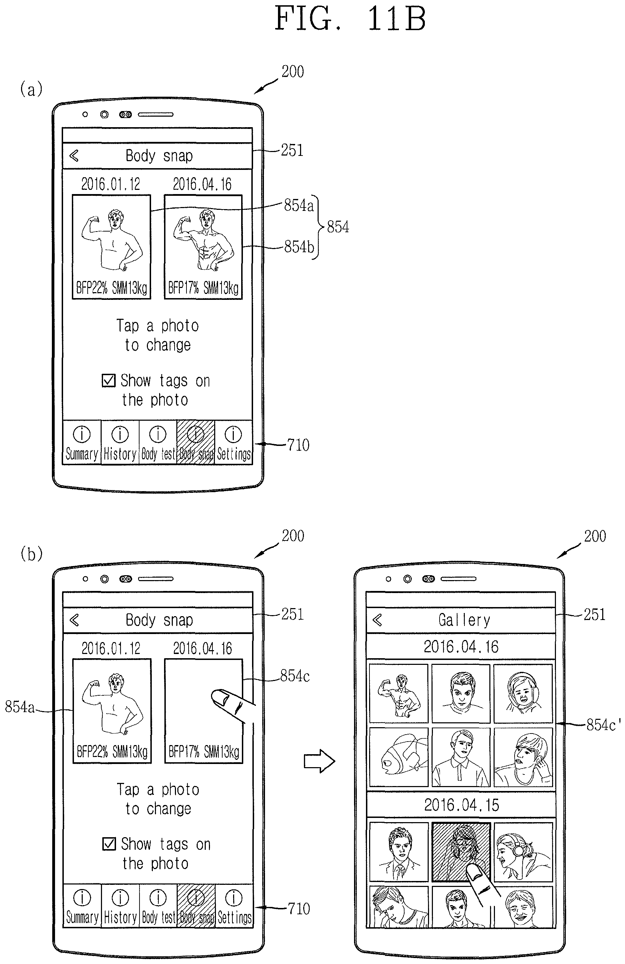

Referring to (a) of FIG. 11B, when the body snap function is executed from the menu image 710, result data 854 may be displayed along with photographic data. The display 251 may display multiple measurement results the body composition measuring device 100 obtained on different dates. The display 251 displays a first photograph 854a and the date and result data for the first photograph 854a. Also, the display 251 displays a second photograph 854b associated with another measurement result and the date and result data for the second photograph 854b.

Referring to (b) of FIG. 11B, if there is no photographic data corresponding to a certain measurement result, the controller 280 controls the display 251 to only display the measurement result. The controller 280 can add photographic data based on touch input on a photograph area 854c of the display 251 where the photographic data is to be displayed. Based on touch input on the photograph area 854c, the controller 280 can execute an application which is configured to include photographic data and work in conjunction with the body composition measuring device 100.

The display 251 displays an execution screen 854c' of the application. The controller 280 outputs an image selected from the execution screen 854c' to the photograph area 854c, and controls the memory 270 to store the selected image along with the result data corresponding to the photograph area 854c.

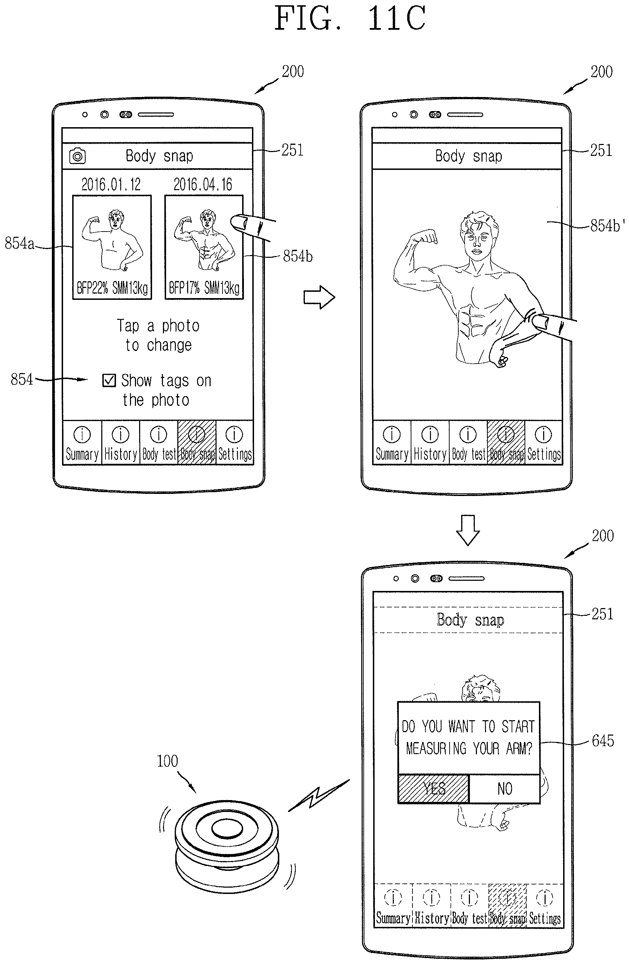

Referring to FIG. 11C, the controller 280 can activate the camera 121 and execute a capture application, based on touch input on the camera icon included in the fifth measurement result screen 850. The display 251 switches from the fifth measurement result screen 850 to the execution screen of the capture application that includes a preview image 854d' captured by the camera 121. The controller 280 obtains a photograph based on a control command applied to the execution screen. The obtained photograph 854e is tagged on the most recent body composition measurement result and displayed on the fifth measurement result screen 850.

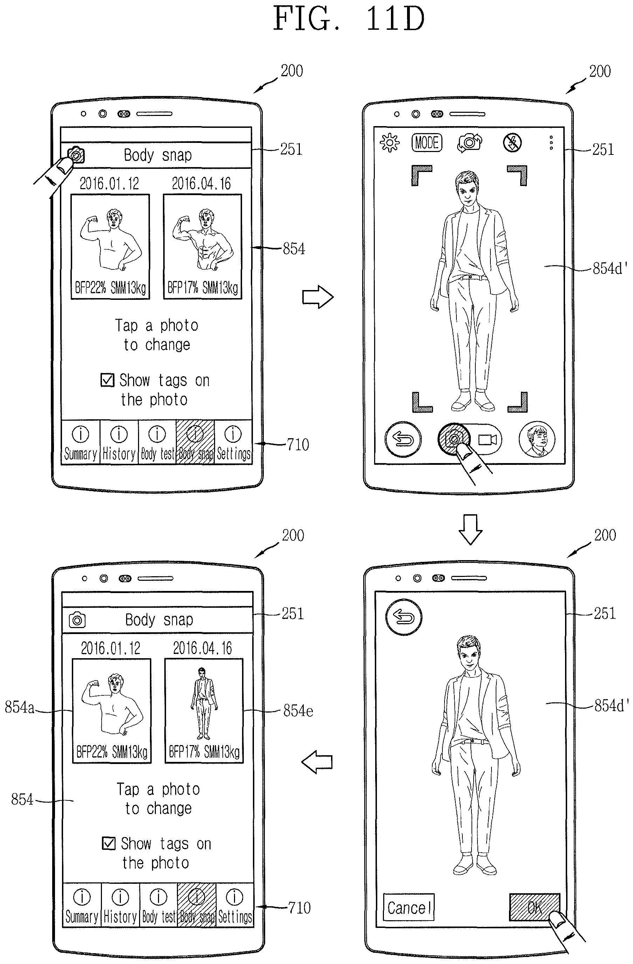

Referring to FIG. 11D, the second photograph 854b is fully displayed on the display 3251 based on a touch on the second photograph 254b. The controller 280280 can select an area of the human body based on a particular type of touch input on the second photograph 854b. For example, when the particular type of touch input is applied to an arm part on the second photograph 854b, the controller 280 can generate a control signal for measuring an arm of the user.

When a part of the body is selected, the controller 280 controls the display 251 to display a confirmation window 645 for confirming whether to measure the selected part of the body. Through the confirmation window 645, the controller 280 can control the wireless communication part 210 to send the control signal to the body composition measuring device 100. Accordingly, if the user wants to get a body composition result while a photograph is on-screen, they may select a part of the human body to intuitively obtain measurement data.

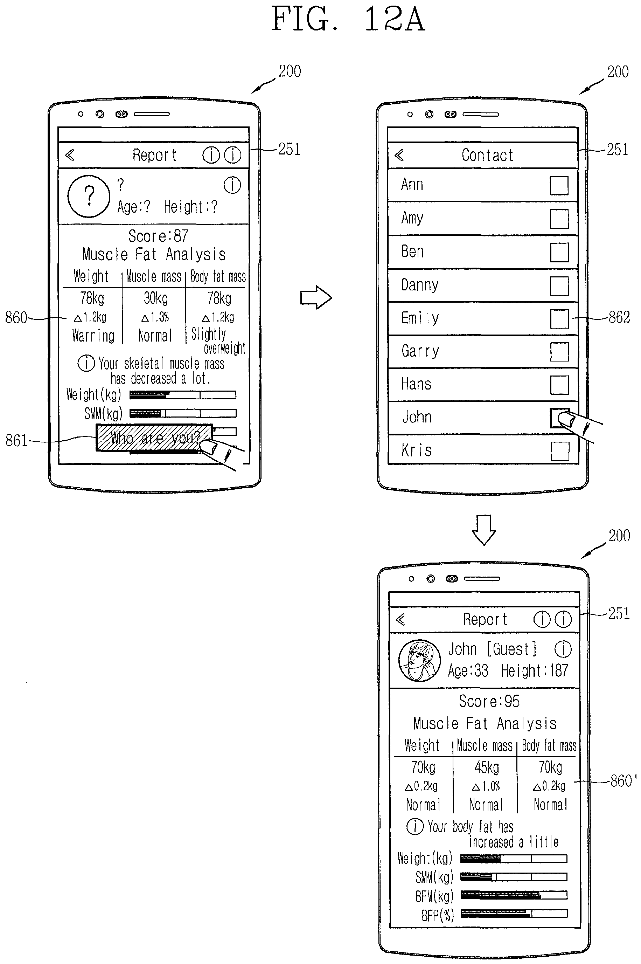

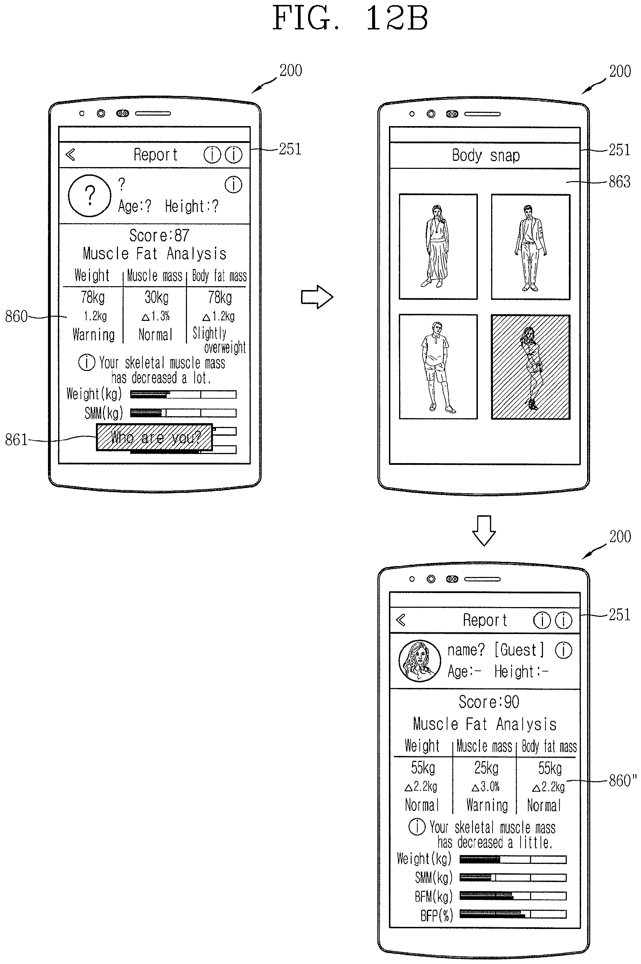

FIGS. 12A and 12B are conceptual diagrams explaining a control method for identifying a person for whom the body composition measuring device 100 has obtained a measurement result. Referring to FIG. 12A, the controller 280 outputs a sixth measurement result screen 860 by using measurement data obtained by the body composition measuring device 100. If no user profile has been set during operation of the body composition measuring device 100, the control part compares the current measurement data obtained by the body composition measuring device 100 with previous measurement data stored along with user profiles. If the current measurement data and the previous measurement data do not match, the controller 280 controls the display 251 to display a graphic image 861 to show user profiles.

Further, if measurement data for a given user, obtained by the body composition measuring device 100, does not match the previous measurement data, the controller 280 controls the display 251 to display the graphic image 861. This means that the difference between the current measurement data and the previous measurement data is greater than a threshold and the given user does not match any of the users who have their body composition measured.

The controller 280 controls the display 251 to display a user list 762 based on touch input on the graphic image 861. For example, the user list 862 may be a list of contacts stored in the memory 270 of the mobile terminal 200. The controller 280 applies the profile of a user selected from the user list 862 to the measurement data, and outputs a sixth measurement result screen 860 containing the user's profile. If the user profile is changed, analysis data obtained using the user profile and the measurement data may be changed. If there is no user profile stored, the controller 280 can display an input window for adding a user profile.

Referring to FIG. 12B, if there is no given user who matches measurement data, the controller 280 extracts an image of a user who is assumed to be the matching user The controller 280 can extract a photograph of a person who matches some of the measurement data from among images stored in the memory 270 or from among photographs captured while the body composition measuring application is active.

The controller 280 controls the display 251 to display screen information 863 including a plurality of photographs. The screen information 863 may include at least one photograph of a user. One of the photographs of a user is selected based on a control command on the screen information 863, and a measurement result screen 860 in which the user profile of the selected photograph is applied to the measurement result is displayed.

According to the above embodiments, when measurement data is created by operating the body composition measuring device 100 without selecting a user profile, or when a designated user profile does not match the created measurement data, the user who correctly matches the measurement data may be selected.

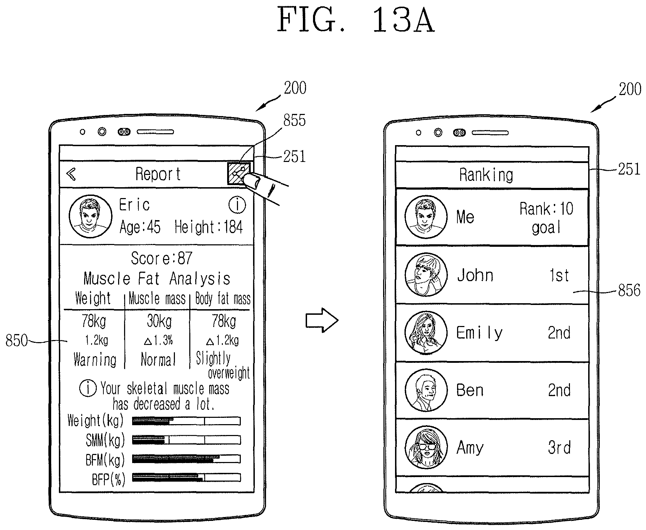

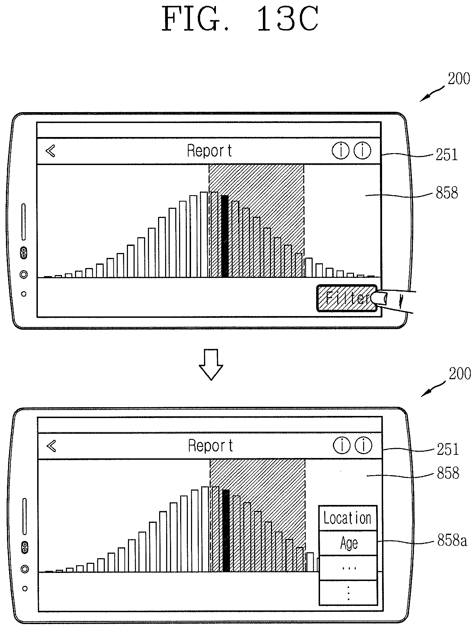

FIGS. 13A and 13C are conceptual diagrams explaining a control method for sharing measurement data. Referring to FIG. 13A, the fifth measurement result screen 850 includes a share icon 855 for sharing measurement data. At least part of the measurement data included in the fifth measurement result screen 850 may be uploaded onto a particular server based on touch input on the share icon 855.

The particular server may receive measurement data corresponding to a plurality of user profiles, set an order of priority according to particular criteria, and provide each user with the order-of-priority information. A page 856 for the particular server may show the priority of the user of the mobile terminal 200. Accordingly, the user can work towards their goal while competing with the users of other mobile terminals and share their body information, images, etc. with other others.

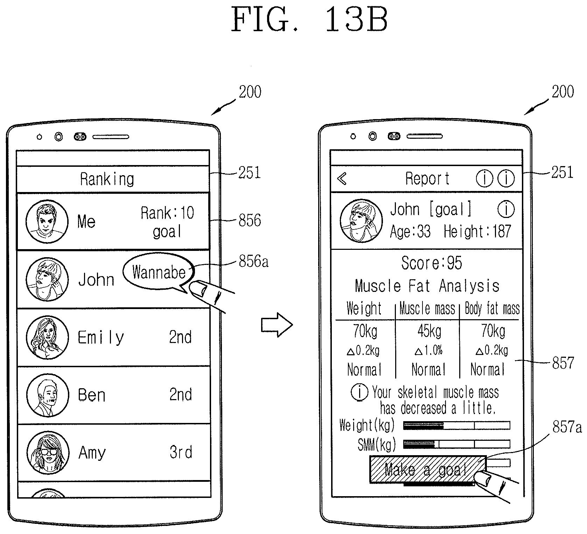

Referring to FIG.13B, the controller 280 can create target data using the page 856 for the particular server. When touch input is applied to the body measurement data of a particular user included in the page 856, the controller 280 can control the display 251 to display a goal setting icon 856a. When a touch is applied to the target setting icon 856a, the controller 280 receives selected measurement data. The controller 280 sets the selected measurement data as the user's target data.

The controller 280 compares the received measurement data with the user's measurement data to create variation data 857 for the target data, and outputs it on a measurement result screen. The controller 280 can select target data using another user' measurement data based on a touch on a graphic image 857a, which is output along with the variation data 957, and may access the server and continuously monitor the selected measurement data of the another user. Accordingly, the mobile terminal may find a proper user that is matched the measurement result, and then the user does not need to select user information.

FIG. 13C is a conceptual diagram explaining a method of providing a screen of an analysis result of shared measurement data. Referring to FIG. 13C, the mobile terminal 200 may create a graph of distribution of one substance (e.g., body fat) of the body composition by using measurement data shared through the wireless communication part. The display displays an analysis result screen 858 including the distribution graph. The display 251 of the mobile terminal 200 displays user data on the distribution graph. Accordingly, the user can check their ranking in body composition among the people whom the user shares body composition data.

Further, the controller 280 can take some of the shared data that meets with a particular criterion and calculate measurement data. The analysis result screen 858 includes a graphic image, and the display 251 displays a reference list for selecting part of the shared data based on a touch on the graphic image.

For example, the controller 280 can selectively output measurement data based on at least one of the following variables, including the age, gender, height, race, location, etc. of the user corresponding to the body composition result. In this instance, a distribution graph may be created based on the extracted, and the user's body composition data may be displayed on the distribution graph. Accordingly, the user can check the distribution of their body composition relative to the average distribution for collected data.

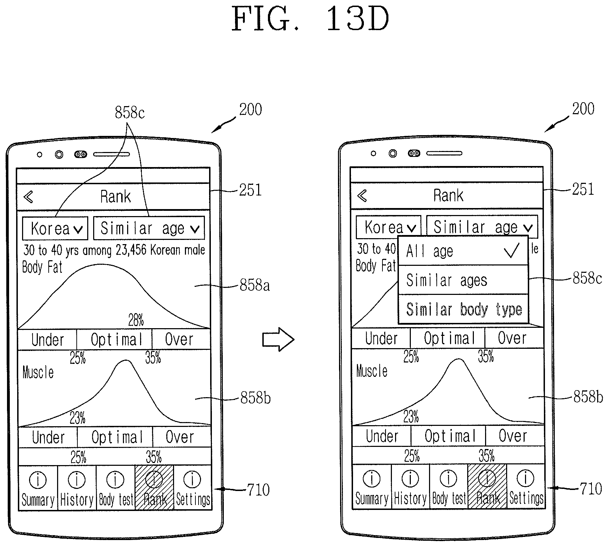

FIG. 13D is a conceptual view illustrating a control method to display a distribution graph in a filtering manner. Referring to FIG. 13D, an analysis result screen 858 includes a first graph 858a related to a body fat, and a second graph 858b related to a muscle mass. The first and second graphs 858a, 858b may be output together.

The analysis result screen 858 includes a graphic image 858c for inputting a filtering condition. The filtering condition includes an age, a nationality, a body type, etc. The filtering condition is applied to the first and second graphs 858a, 858b. Thus, a user can check data on his or her body, through users who belong to a specific condition.

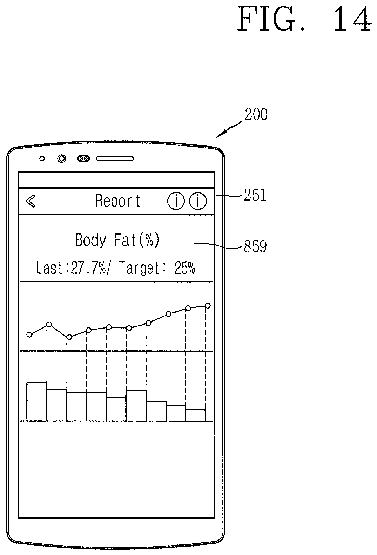

FIG. 14 is a conceptual diagram explaining a method of controlling a body composition measurement and motion data according to one embodiment. The memory of the mobile terminal 200 stores body fat data, which is part of a body composition measurement result, over time. Also, the sensing part equipped in the mobile terminal 200 senses a user's motion. Motion data sensed by the sensing part is stored in the memory over time.

The controller 280 controls the display 251 to display a graph representing the received body fat data. Also, the controller 280 extracts motion data, which is obtained for a period of time between dates of measurement of the body fat data, and outputs it on the display 251. The motion data displayed on the display 251 may be an average value.

The display 251 displays comparison screen information 859 including a first graph representing body fat data by date and a second graph representing average motion data. The first and second graphs are of the same date. Consequently, even if the body composition data is measured on an irregular basis, the user can see a body composition measurement result in connection with the amount of exercise since motion data, obtained for a period of time between dates of measurement, is displayed.

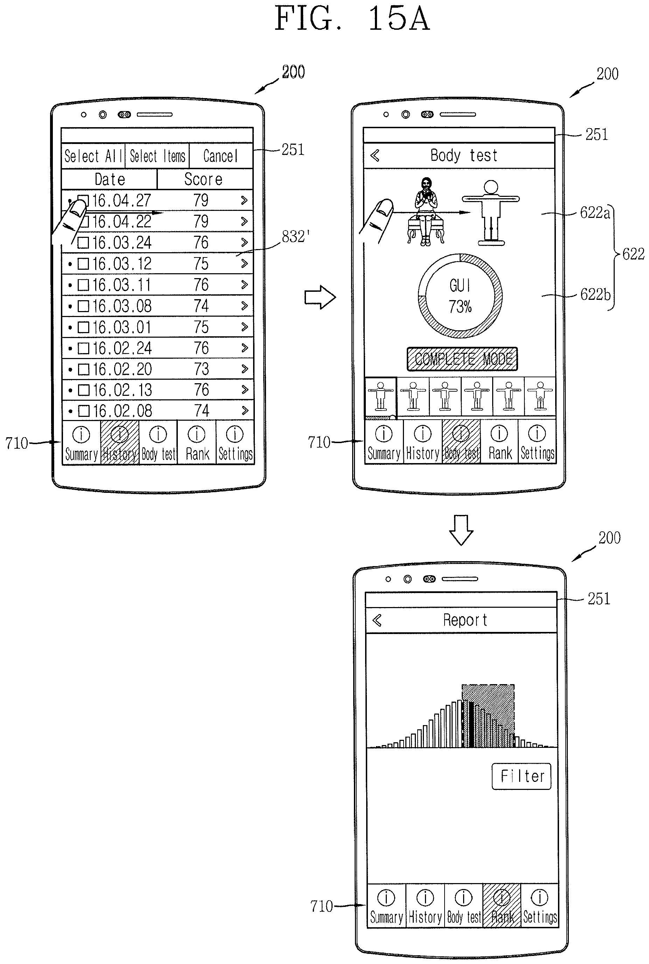

FIGS. 15A to 15B are conceptual views illustrating a method to control an application execution screen. Referring to FIG. 15A, the application execution screen includes the menu image 710. The menu image 710 includes a summary icon which provides user information or outputs guide information, a history icon which outputs various data on pre-stored measurement results, a body test icon which measures data on a body composition, a rank icon which outputs an image stored (to be stored) together with the data on a body composition, and a setting icon for setting a driving of the body composition measuring device.

The display unit 180 outputs screen information corresponding to an icon selected among a plurality of icons included in the menu image 710. For instance, if the history icon is selected, the list screen 832' (refer to FIG. 9B) is output. The controller 280 can select another icon, based on a touch input applied to a dragging type of touch input applied to the list screen 832'. If the history icon is converted into the body test icon (measurement icon), the display unit outputs the second measurement screen 622 (refer to FIG. 5B). When a dragging type of touch input is applied to the second measurement screen 622, a ranking icon indicating a distribution of measured data may be selected. That is, a user can select a desired menu by applying a touch input in a specific direction.

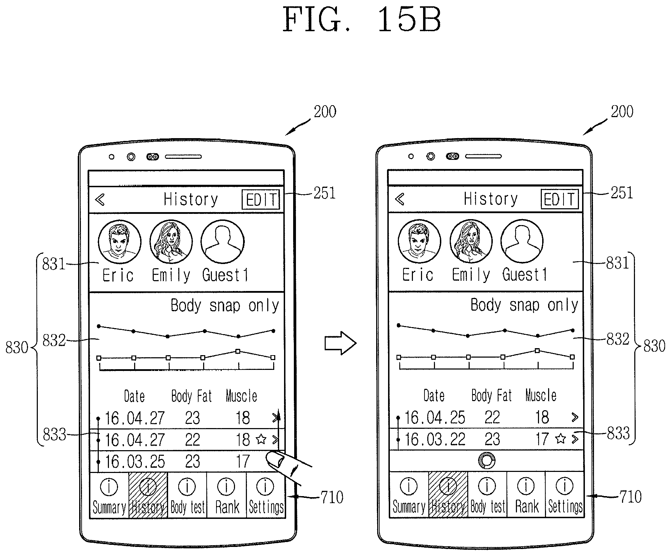

Referring to FIG. 15B, the third measurement result screen 830 includes the user profiles 831 of multiple users, the result page 832 for a selected user, and history information 833. The user profiles 831 of multiple users, the result page 832 for a selected user, and the history information 833 may be sequentially disposed on divided regions of the display unit 151. Based on a touch input applied to the history information 833, the controller 280 controls the display unit 151 to output additional information included in the history information 833. Here, information included in the user profiles 831 of multiple users and the result page 832 for a selected user, is not changed.

In addition, when a touch input is applied to each of the user profiles 831 of multiple users, the result page 832 for a selected user, and the history information 833, only information related to the touched region is changed. Thus, a user can be provided with additional information, in a state where a plurality of different information is displayed on a single page.

Various embodiments may be implemented using a machine-readable medium having instructions stored thereon for execution by a processor to perform various methods presented herein. Examples of possible machine-readable mediums include HDD (Hard Disk Drive), SSD (Solid State Disk), SDD (Silicon Disk Drive), ROM, RAM, CD-ROM, a magnetic tape, a floppy disk, an optical data storage device, the other types of storage mediums presented herein, and combinations thereof. If desired, the machine-readable medium may be realized in the form of a carrier wave (for example, a transmission over the Internet). The processor may include a controller of the mobile terminal.

## Claims

1. A body composition measuring device comprising:
a body portion including first and second sides facing each other and a lateral side connecting the first and second sides;
a first electrode portion provided on the first side of the body portion;
a second electrode portion on the second side of the body portion; and
a control part that measures an impedance when the first and second electrode portions come into contact with first and second areas of a user holding the body composition measuring device.

2. The body composition measuring device of claim 1, wherein the body portion includes an insulating material, and
wherein the first and second electrode portions each comprise first and second electrodes spaced apart from each other.

3. The body composition measuring device of claim 2, wherein the first and second electrodes included in the first and second electrode portions are symmetrical.

4. The body composition measuring device of claim 3 or 4, wherein the first and second electrode portions protrude from the first and second sides.

5. The body composition measuring device of any one of claims 2 to 4, wherein the second electrode is located at a center and the first electrode surrounds the second electrode.

6. The body composition measuring device of any one preceding claim, wherein at least part of the first and second sides has a curved surface that bulges outward.

7. The body composition measuring device of any one of claims 2 to 6, further comprising:
a circuit board located inside the body portion and including the control part; and
a connecting portion that extends into the body portion from the first and second electrodes and that is electrically connected to the circuit board.

8. The body composition measuring device of any one preceding claim, wherein the body portion comprises:
first and second bodies respectively comprising the first and second sides facing each other; and
a third body connecting the first and second bodies, and
wherein a cross-section of the third body is narrower than a cross-section of the first and second bodies.

9. The body composition measuring device of claim 8, further comprising:
a length measuring portion on the first and second bodies and exposed to an outside by an external force to enable a measurement of a length and circumference of a part of a body of the user.

10. The body composition measuring device of any one preceding claim, further comprising:
a sensing part mounted in an area of the body portion and sensing a rotation and movement of the body portion,
wherein a center of the first and second sides of the body portion includes a recess, and part of the first and second electrode portions overlaps the recess, and
wherein the control part calculates a measurement result of a length of a part of a body of the user based on a rotation of the body portion relative to an axis of rotation at the recess.

11. The body composition measuring device of any one preceding claim, further comprising:
a wireless communication part that performs wireless communication with a particular external device,
wherein the control part controls the wireless communication part to send an impedance measurement or measurement data calculated from the impedance to the particular external device.

12. The body composition measuring device of claim 11, wherein, in a simple mode, the control part obtains an impedance measurement corresponding to a single posture while the first and second electrode portions are in contact with a body of the user, and in a complete mode, the control part obtains a plurality of impedance measurements corresponding to a plurality of postures while the first and second electrode portions are in contact with the body of the user.

13. The body composition measuring device of claim 11 or 12, wherein the control part calculates a body composition measurement result by adding the plurality of impedance measurements obtained in the complete mode or subtracting the impedance measurement of a redundant area.
